(19) Europäisches Patentamt
European Patent Office
Office européen des brevets

(11) EP 0 949 261 A1

(12) # EUROPEAN PATENT APPLICATION
published in accordance with Art. 158(3) EPC

(43) Date of publication:
**13.10.1999 Bulletin 1999/41**

(21) Application number: **97950390.1**

(22) Date of filing: **25.12.1997**

(51) Int. Cl.[6]: **C07D 413/10**, C07D 417/10, C07D 401/10, A01N 43/40, A01N 43/76, A01N 43/80, A01N 43/78, A01N 43/836, A01N 43/824, A01N 43/713, A01N 43/56, A01N 43/653

(86) International application number:
**PCT/JP97/04815**

(87) International publication number:
**WO 98/29412 (09.07.1998 Gazette 1998/27)**

(84) Designated Contracting States:
**DE FR GB IT**

(30) Priority: **27.12.1996 JP 35852096**
**29.10.1997 JP 31275097**

(71) Applicant: **NIPPON SODA CO., LTD.**
**Chiyoda-ku, Tokyo 100-8165 (JP)**

(72) Inventors:
- **ADACHI, Hiroyuki**
  **Odawara Research Center**
  **Kanagawa 250-02 (JP)**
- **SAGAE, Takahiro**
  **Odawara Research Center**
  **Kanagawa 250-02 (JP)**
- **KOGUCHI, Masami**
  **Odawara Research Center**
  **Kanagawa 250-02 (JP)**
- **KAWANA, Takashi**
  **Odawara Research Center**
  **Kanagawa 250-02 (JP)**

(74) Representative:
**Schmitz, Jean-Marie et al**
**Dennemeyer & Associates Sàrl**
**P.O. Box 1502**
**1015 Luxembourg (LU)**

(54) **SUBSTITUTED PIPERIDINEDIONE DERIVATIVES AND HERBICIDES**

(57) Substituted piperidinedione derivatives represented by a general formula (1) or salts thereof and herbicides containing one or more of these compounds;

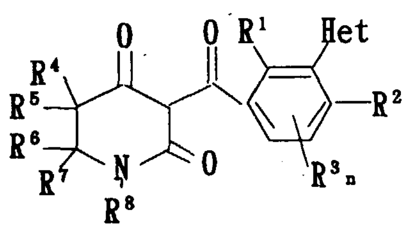

(1)

wherein $R^1$ represents halogeno, $C_{1-6}$ alkyl, etc. ; $R^2$ represents halogeno, $C_{1-6}$ alkylsulfonyl, etc. ; Het represents a saturated or unsaturated five-membered heterocycle containing one to four N, O or S atoms, substituted with hydrogen, $C_{1-6}$ alkyl, etc., and is bonded to the benzene ring at the carbon atom thereof; and $R^4$, $R^5$, $R^6$, $R^7$ and $R^8$ each represents hydrogen, $C_{1-6}$ alkyl, etc.

EP 0 949 261 A1

## Description

Field of the Invention

The present invention is related to substituted piperidine derivatives wherein a benzoyl group is substituted at the third position of the piperidine ring, and to herbicides containing one or more of these piperidine derivatives.

Background Art

In recent years, many herbicides have been used for weed control in agricultural and horticultural crop cultivation which is requiring great numbers of labors. However, due to causing several problems including phytotoxicity on crops, residues and pollution in the environment, herbicides which has firm effectiveness with a lower dose and is usable in safe way from various points of view are badly required in the industry.

As a related-technology to the present invention, cyclohexanedione compounds represented by the following general formula having a herbicidal activity;

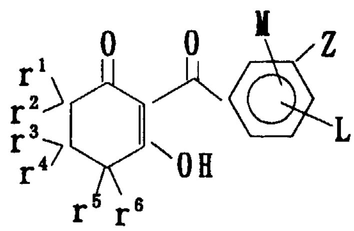

wherein $r^1$ to $r^6$ each represents hydrogen, $C_{1-6}$ alkyl, etc., L and M each represents hydrogen, halogeno, $SO_2R$, etc., and Z represents a 5-or 6-membered heterocycle, are disclosed in International Patent Open No. WO96/26200 Gazette.

Whereas, pyrazole compounds represented by the following general formula and each having herbicidal activity;

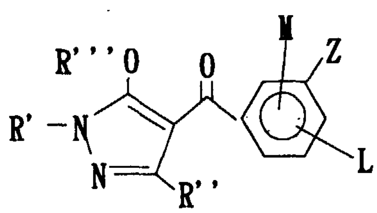

wherein R' and R'' each represents hydrogen, $C_{1-6}$ alkyl, etc., R''' represents hydrogen, phenylsulfonyl, etc., L and M each represents hydrogen, halogeno, $SO_2R$, etc. , and Z represents a 5- or 6-membered heterocycle, are disclosed in International Patent Open No. WO96/26206 Gazette.

It is an object of the present invention to provide herbicides which can be advantageously manufactured in an industrial scale, has firm effectiveness with a lower dose, is highly safe, and has high non-harmful selectivity to agricultural and horticultural crops.

Disclosure of the Invention

The present invention is directed to substituted piperidinedione derivatives represented by a general formula (1) or salts thereof;

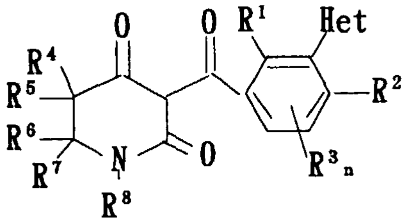

(1)

wherein $R^1$ represents nitro, cyano, halogeno, $C_{1-6}$ alkyl, $C_{1-6}$ alkoxy, $C_{1-6}$ haloalkyl, $C_{1-6}$ alkylthio, $C_{1-6}$ alkylsulfinyl or $C_{1-6}$ alkylsulfonyl;

$R^2$ represents nitro, cyano, halogeno, $C_{1-6}$ alkyl, $C_{1-6}$ alkoxy, $C_{1-6}$ haloalkyl, $C_{1-6}$ haloalkoxy, $C_{1-6}$ alkylthio, $C_{1-6}$ alkylsulfinyl or $C_{1-6}$ alkylsulfonyl;

$R^3$ represents nitro, cyano, halogeno, $C_{1-6}$ alkyl, $C_{1-6}$ alkoxy, $C_{1-6}$ haloalkyl, $C_{1-6}$ alkylthio, $C_{1-6}$ alkylsulfinyl or $C_{1-6}$ alkylsulfonyl;

n is 0, 1 or 2;

$R^4$, $R^5$, $R^6$ and $R^7$ each independently represents hydrogen, halogeno, $C_{1-6}$ alkyl, $C_{1-6}$ haloalkyl, $C_{1-6}$ alkoxy $C_{1-6}$ alkyl, $C_{1-6}$ alkylcarbonyloxy $C_{1-6}$ alkyl, or hydroxy $C_{1-6}$ alkyl; or $R^4$ and $R^5$, or $R^6$ and $R^7$ may form in together a $C_{2-5}$ alkylene chain, or, $R^5$ and $R^6$ may form in together a single bond or $C_{1-4}$ alkylene chain;

$R^8$ represents hydrogen, $C_{1-6}$ alkyl, $C_{2-6}$ alkenyl, $C_{2-6}$ alkynyl, $C_{1-6}$ haloalkyl, $C_{2-6}$ haloalkenyl, $C_{2-6}$ haloalkynyl, $C_{1-6}$ alkoxy, $C_{2-6}$ alkenyloxy, $C_{2-6}$ alkylnyloxy, $C_{1-6}$ haloalkoxy, $C_{2-6}$ haloalkenyloxy, or $C_{2-6}$ haloalkynyloxy;

Het represents a saturated or unsaturated 5-membered heterocycle substituted with $R^9$ and $R^{10}$ which contains 1 to 4 N, O or S atoms, and Het is bonded to benzene ring at the carbon atom;

$R^9$ and $R^{10}$ each independently represents hydrogen, halogeno, $C_{1-6}$ alkyl or $C_{1-6}$ alkoxy, and herbicides containing one or more of said derivatives as an active ingredient.

Now, the present invention is described in detail in the following.

As examples for a group represented by $R^1$ in the general formula (1), halogeno, such as fluorine, chlorine and bromine, $C_{1-6}$ alkyl, such as methyl, ethyl, propyl, isopropyl, butyl, isobutyl and t-butyl, $C_{1-6}$ alkoxy, such as methoxy, ethoxy, propoxy, isopropoxy, butoxy and t-butoxy, $C_{1-6}$ haloalkyl, such as trifluoromethyl, trichloromethyl, fluoromethyl, chloromethyl, difluoromethyl, dichloromethyl, trifluoroethyl and pentafluoroethyl, $C_{1-6}$ alkylthio, such as methylthio, ethylthio, propylthio and isopropylthio, $C_{1-6}$ alkylsulfinyl, such as methylsulfinyl, ethylsulfinyl, propylsulfinyl and isopropylsulfinyl, $C_{1-6}$ alkylsulfonyl, such as methylsulfonyl, ethylsulfonyl, propylsulfonyl and isopropylsulfonyl, can be given.

As examples for a group represented by $R^2$ in the general formula (1), halogeno, such as fluorine, chlorine and bromine, $C_{1-6}$ alkyl, such as methyl, ethyl, propyl, isopropyl, butyl, isobutyl and t-butyl, $C_{1-6}$ alkoxy, such as methoxy, ethoxy, propoxy, isopropoxy, butoxy and t-butoxy, $C_{1-6}$ haloalkyl, such as trifluoromethyl, trichloromethyl, fluoromethyl, chloromethyl, difluoromethyl, dichloromethyl, trifluoroethyl and pentafluoroethyl, $C_{1-6}$ haloalkoxy, such as trifluoromethoxy and trichloromethoxy, $C_{1-6}$ alkylthio, such as methylthio, ethylthio, propylthio and isopropylthio, $C_{1-6}$ alkylsulfinyl, such as methylsulfinyl, ethylsulfinyl, propylsulfinyl and isopropylsulfinyl, $C_{1-6}$ alkylsulfonyl, such as methylsulfonyl, ethylsulfonyl, propylsulfonyl and isopropylsulfonyl, can be given.

As examples for a group represented by $R^3$ in the general formula (1), halogeno, such as fluorine, chlorine and bromine, $C_{1-6}$ alkyl, such as methyl, ethyl, propyl, isopropyl, butyl, isobutyl and t-butyl, $C_{1-6}$ alkoxy, such as methoxy, ethoxy, propoxy, isopropoxy, butoxy and t-butoxy, $C_{1-6}$ haloalkyl, such as trifluoromethyl, trichloromethyl, fluoromethyl, chloromethyl, difluoromethyl, dichloromethyl, trifluoroethyl and pentafluoroethyl, $C_{1-6}$ alkylthio, such as methylthio, ethylthio, propylthio and isopropylthio, $C_{1-6}$ alkylsulfinyl, such as methylsulfinyl, ethylsulfinyl, propylsulfinyl and isopropylsulfinyl, $C_{1-6}$ alkylsulfonyl, such as methylsulfonyl, ethylsulfonyl, propylsulfonyl and isopropylsulfonyl, can be given.

Further, as examples for a group represented by $R^4$, $R^5$, $R^6$ and $R^7$ in the general formula (1), halogeno, such as fluorine, chlorine and bromine, $C_{1-6}$ alkyl, such as methyl, ethyl, propyl, isopropyl, butyl, isobutyl and t-butyl, $C_{1-6}$ haloalkyl, such as trifluoromethyl, trichloromethyl, fluoromethyl, chloromethyl, iodomethyl, dichloromethyl, difluoromethyl, trifluoroethyl and pentafluoroethyl, $C_{1-6}$ alkoxy $C_{1-6}$ alkyl, such as methoxymethyl, methoxyethyl, ethoxymethyl and ethoxyethyl, $C_{1-6}$ alkylcarbonyloxy $C_{1-6}$ alkyl, such as methylcarbonyloxymethyl, ethylcarbonyloxymethyl, methylcarbonyloxyethyl and ethylcarbonyloxyethyl, hydroxy $C_{1-6}$ alkyl, such as hydroxymethyl, hydroxyethyl and hydroxypropyl, can be given, respectively. As examples for $C_{2-5}$ alkylene chain, ethylene, trimethylene, tetramethylene, etc. can be given, and as examples for $C_{1-4}$ alkylene chain, methylene, ethylene, trimethylene and the like can be given.

As examples for a group represented by $R^8$ in the general formula (1), $C_{1-6}$ alkyl, such as methyl, ethyl, propyl, isopropyl, butyl, isobutyl and t-butyl, $C_{2-6}$ alkenyl, such as vinyl, 1-propenyl, allyl and 2-butenyl, $C_{2-6}$ alkynyl, such as ethynyl, 1-propynyl and 2-propynyl, $C_{1-6}$ haloalkyl, such as trifluoromethyl, trichloromethyl, fluoromethyl, chloromethyl, difluoromethyl, dichloromethyl, trifluoroethyl and pentafluoroethyl, $C_{2-6}$ haloalkenyl, such as 2-chlorovinyl, 2,2-difluorovinyl, 3-chloroallyl, 3,3-dichloroallyl and 2-chloroallyl, $C_{2-6}$ haloalkynyl, such as 2-chloroethynyl, 3-chloro-2-propynyl and 3-fluoro-2-propynyl, $C_{1-6}$ alkoxy, such as methoxy, ethoxy, propoxy, isopropoxy, butoxy and t-butoxy, $C_{2-6}$ alkenyloxy, such as vinyloxy, 1-propenyloxy, allyloxy and 2-butenyloxy, $C_{2-6}$ alkynyloxy, such as ethynyloxy and 2-propynyloxy, $C_{1-6}$ haloalkoxy, trifluoromethoxy, trichloromethoxy, fluoromethoxy, chloromethoxy, difluoromethoxy, dichloromethoxy, trifluoroethoxy and pentafluoroethoxy, $C_{2-6}$ haloalkenyloxy, such as 2-chlorovinyloxy, 2,2-difluorovinyloxy, 3-chloroallyloxy, 3,3-dichloroallyloxy and 2-chloroallyloxy, $C_{2-6}$ haloalkynyloxy, such as 2-chloroethynyloxy, 3-chloro-2-propynyl and 3-fluoro-2-propynyl, can be given.

Het represents a saturated or unsaturated 5-membered heterocycle containing 1 to 4 atoms of any of N, O and S and substituted with $R^9$ and $R^{10}$, and said heterocycle is bonded to a benzene ring at the carbon atom thereof. As examples for the heterocycle represented by Het, 2-furyl, 3-furyl, oxazole-2-yl, oxazole-4-yl, oxazole-5-yl, isoxazole-3-yl, isoxazole-4-yl, isoxazole-5-yl, 1,2,4-oxadiazole-3-yl, 1,2,4-oxadiazole-5-yl, 1,3,4-oxadiazole-2-yl, 1,2,3-oxadiazole-4-yl, 1,2,3-oxadiazole-5-yl, 1,2,5-oxadiazole-3-yl, thiazole-2-yl, thiazole-4-yl, thiazole-5-yl, isothiazole-3-yl, isothiazole-4-yl, isothiazole-5-yl, 1,2,4-thiazole-3-yl, 1,2,4-thiadiazole-5-yl, 1,3,4-thiadiazole-2-yl, 1,2,3-thiadiazole-4-yl, 1,2,3-thiadiazole-5-yl, 1,2,5-thiadiazole-3-yl, imidazole-2-yl, imidazole-4-yl, imidazole-5-yl, 2-thienyl, 3-thienyl, 2-pyrrolyl, 3-pyrrolyl, pyrazole-3-yl, pyrazole-4-yl, pyrazole-5-yl, 1,2,4-triazole-3-yl, 1,2,4-triazole-5-yl, 1,3,4-triazole-2-yl, 1,2,3-triazole-4-yl, 1,2,3-triazole-5-yl, tetrazole-5-yl and the like can be given.

Further, as examples for the saturated 5-membered hetericycle, 2-tetrahydrofuranyl, 3-tetrahydrofuranyl, 2-tetrahydrothienyl, 3-tetrahydrothienyl, tetrahydrothiopyran-2-yl, tetrahydrothiopyran-3-yl, tetrahydrothiopyran-4-yl, 1,3-dithiane-2-yl, 1,3-dithiane-4-yl, 1,3-dithiolane-2-yl, 1,3-dithiolane-4-yl, 5,6-dihydro-4H-1, 3-thiazine-2-yl, 1,3-oxathiolane-2-yl, 1,3-oxathiane-2-yl, 1-pyrrolidinyl, 2-pyrrolidinyl, 3-pyrrolidinyl, 3-isoxazolidinyl, 4-isoxazolidinyl, 5-isoxazolidinyl, 3-isothiazolidinyl, 4-isothiazolidinyl, 5-isothiazolidinyl, 2-pyrrolidinyl, 3-pyrrolidinyl, 2-oxolydinyl, 4-oxolydinyl, 5-oxolydinyl, 2-thiazolydinyl, 4-thiazolydinyl, 5-thiazolydinyl, 2-imidazolydinyl, 4-imidazolydinyl, 1,2,4-oxadiazolydine-3-yl, 1,2,4-oxadiazolydine-5-yl, 1,3,4-oxadiazolydine-2-yl, 1,3,4-oxadiazolydine-5-yl, 1,2,4-thiadiazolydine-3-yl, 1,2,4-thiadiazolydine-5-yl, 1,3,4-thiadiazolydine-2-yl, 1,3,4-thiadiazolydine-5-yl, 1,3,4-triazolydine-2-yl, 2,3-dihydrofuran-2-yl, 2,3-dihydrofuran-3-yl, 2,4-dihydrofuran-3-yl, 2,3-dihydrothiene-2-yl, 2,3-dihydrothiene-3-yl, 2,4-dihydrothiene-2-yl, 2,4-dihydrothiene-3-yl, 2,3-pyrroline-2-yl, 2,3-pyrroline-3-yl, 2,4-pyrroline-2-yl, 2,4-pyrroline-3-yl, 2,3-isoxazoline-3-yl, 3,4-isoxazoline-3-yl, 4,5-isoxazoline-3-yl, 2,3-isoxazoline-4-yl, 3,4-isoxazoline-4-yl, 4,5-isoxazoline-4-yl, 2,3-isoxazoline-5-yl, 3,4-isoxazoline-5-yl, 4,5-isoxazoline-5-yl, 2,3-isothiazoline-3-yl, 3,4-isothiazoline-3-yl, 4,5-isothiazoline-3-yl, 2,3-isothiazoline-4-yl, 3,4-isothiazoline-4-yl, 4,5-isothiazoline-4-yl, 2,3-isothiazoline-5-yl, 3,4-isothiazoline-5-yl, 4,5-isothiazoline-5-yl, 2,3-dihydropyrrazole-1-yl, 2,3-dihydropyrrazole-2-yl, 2,3-dihydropyrrazole-3-yl, 2,3-dihydropyrrazole-4-yl, 2,3-dihydropyrrazole-5-yl, 3,4-dihydropyrrazole-1-yl, 3,4-dihydropyrrazole-2-yl, 3,4-dihydropyrrazole-3-yl, 3,4-dihydropyrrazole-4-yl, 3,4-dihydropyrrazole-5-yl, 4,5-dihydropyrrazole-1-yl, 4, 5-dihydropyrrazole-2-yl, 4,5-dihydropyrrazole-3-yl, 4,5-dihydropyrrazole-4-yl, 4,5-dihydropyrrazole-5-yl, 2,3-dihydroxazole-2-yl, 2,3-dihydroxazole-3-yl, 2,3-dihydroxazole-4-yl, 2,3-dihydroxazole-5-yl, 4,5-dihydroxazole-2-yl, 4,5-dihydroxazole-3-yl, 4,5-dihydroxazole-4-yl, 4,5-dihydroxazole-5-yl, 1,3-dioxolane-2-yl, 1,3-dioxolane-4-yl, 1,3-dioxolane-5-yl, 1,4-dioxolane-2-yl and the like can be given.

Whereas, these heterocycles may contain at an optional position substituents, $R^9$ and $R^{10}$, for examples, halogeno, such as fluorine, chlorine and bromine, $C_{1-6}$ alkyl, such as methyl and ethyl, $C_{1-6}$ alkoxy, such as methoxy and ethoxy, and $C_{1-6}$ haloalkyl, such as trifluoromethyl.

As preferable examples for Het, the following heterocycle can be shown.

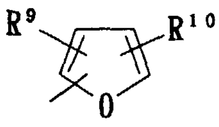 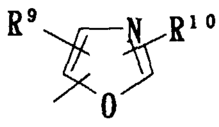 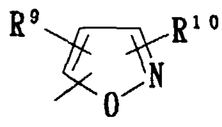

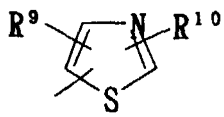 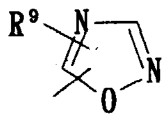 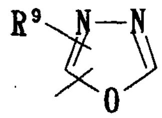

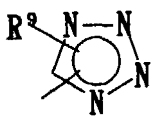 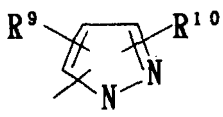 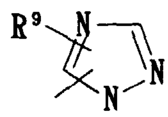

wherein $R^9$ and $R^{10}$ are as defined above.

Further, as more preferable examples for the heterocycles, the following from A to N, can be given.

A : 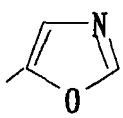 B : 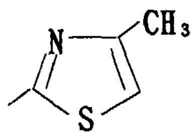 C : 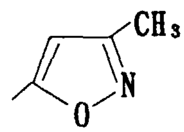

D : 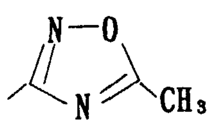 E : 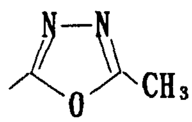 F : 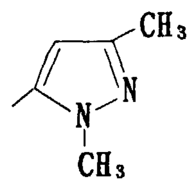

G : 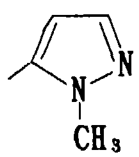 H : 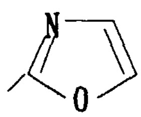 I : 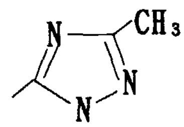

J : 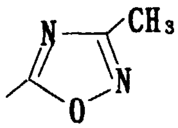 K : 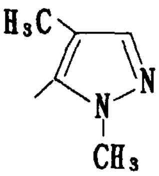 L : 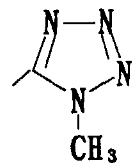

M : 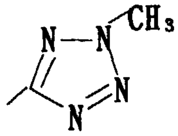 N : 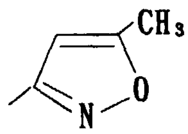

As to the inventive compounds and the intermediate compounds as shown below, the numbers of carbon atoms in the substituents may be in a range of from 1 to 6 as alkyl or from 2 to 6 as alkenyl or alkynyl, and more preferably from 1 to 4 as alkyl or from 2 to 4 as alkenyl or alkynyl.

The compounds of the present invention can be manufactured according to the methods described below.

Manufacturing Method (i)

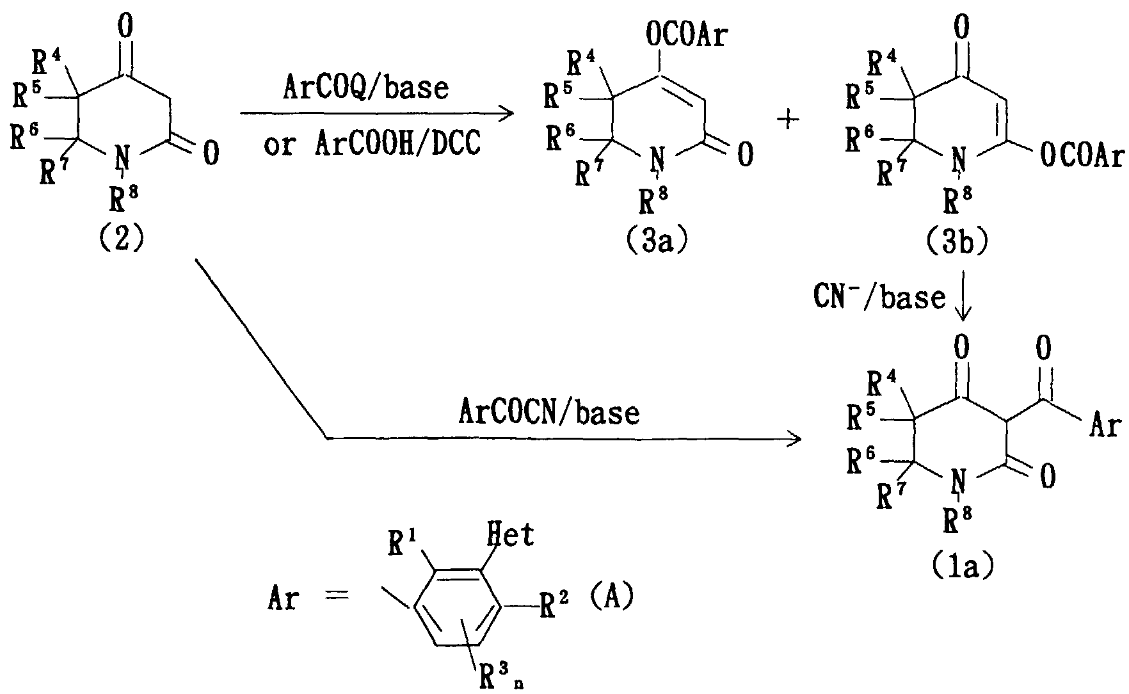

(2) (3a) + (3b) (1a)

Ar = (A)

In the reaction formula as shown above, $R^1$, $R^2$, $R^3$, $R^4$, $R^5$, $R^6$, $R^7$ and $R^8$, and n and Het are as defined above, Q represents halogeno, alkyl carbonyloxy, alkoxy carbonyloxy or benzoyloxy, and Ar represents a group A which is defined in the above.

In the manufacturing process shown above, a compound (3a) and a compound (3b) are obtainable by reacting with a compound (2) in an amount of 1 mole and ArCOQ in an amount of 1 mole, wherein Ar and Q are as defined above, or either of the compound (2) or ArCOQ in an excess amount may be used in the reaction, in the presence of a base in an amount of either 1 mole or an excess amount. As examples for the base usable for the reaction above, an alkali metal hydroxide, such as KOH and NaOH, an alkali metal carbonate, such as sodium carbonate and potassium

carbonate, an alkaline earth metal hydroxide, such as calcium hydroxide and magnesium hydroxide, an alkaline earth metal carbonate, such as calcium carbonate, a tri($C_{1-6}$)alkylamine, such as triethylamine and diisopropylethylamine, an organic base, such as pyridine, sodium phosphate and the like can be given.

As examples for a solvent usable in the process above, $H_2O$, methylene chloride, chloroform, toluene, ethyl acetate, N,N-dimethylformamide (DMF), tetrahydrofuran (THF), dimethoxyethane (DME), acetonitrile and the like can be given.

The mixture prepared for the reaction was stirred at a temperature ranging from 0 to 50°C until completion of the reaction. In addition, the reaction can be proceeded in two phase system by using a phase transfer catalyst, such as quaternary ammonium salt.

Moreover, the compounds (3a) and (3b) are also obtainable by allowing the compound (2) to a reaction with ArCOOH in the presence of a dehydrating and condensing agent, such as dicyclohexylcarbodiimide (DCC). As examples for a solvent usable in a reaction with DCC, methylene chloride, chloroform, toluene, ethyl acetate, DMF, THF, DME, acetonitrile and the like can be given. The mixture prepared for the reaction are stirred under a temperature ranging from -10 to 50 °C until completion of the reaction. The mixture for the reaction can be processed according to a customarily-known method.

The compounds (3a) and (3b) are used in a rearrangement reaction described hereinbelow. The rearrangement reaction is carried out in the presence of a cyano compound and a mild base. The compound (3a) and the compound (3b) in an amount of 1 mole are allowed to a reaction with a base in an amount of from 1 to 4 mole, preferably from 1 to 2 mole, and a cyano compound in an amount of from 0.01 to 1.0 mole, more preferably from 0. 05 to 0. 2 mole, to obtain a compound (1a). Any of the base as described above can be used as the base to be used in this reaction. As the cyano compound, potassium cyanide, sodium cyanide, acetone cyanohydrine, hydrogen cyanide, a polymer holding potassium cyanide and the like can be used. By adding a small amount of a phase transition catalyst, such as crown ether, the reaction can be completed within a shorter time. The reaction is proceeded under a temperature lower than 80°C, and preferably in a range of from 20 to 40°C. As a solvent usable in this reaction, 1,2-dichloroethane, toluene, acetonitrile, methylene chloride, ethyl acetate, DMF, methylisobutylketone, THF, DME and the like are given as an example.

In addition, the compound (1a) can be prepared by allowing the compound (2) to a reaction with ArCOCN in the presence of a base and a Lewis acid, if appropriate, according to the process as described below.

As examples for the base, an alkali metal hydroxide, such as KOH and NaOH, an alkaline earth metal hydroxide, such as magnesium hydroxide and calcium hydroxide, tri($C_{1-6}$ alkyl)amine, such as triethylamine and diisopropylethylamine, an organic base, such as pyridine, sodium carbonate, sodium phosphate and the like can be given. As an appropriate Lewis acid mentioned above, zinc chloride, aluminium trichloride and the like can be used, but it is preferable to use zinc chloride.

The reaction is proceeded in an organic solvent, such as acetonitrile and methylene chloride, at an appropriate temperature in a range of from -20 °C to a boiling point of the solvent used.

Manufacturing Method (ii)

Further, an objective compound (1b) is also obtainable by using the compound (1a) prepared as described above according to the method in the following;

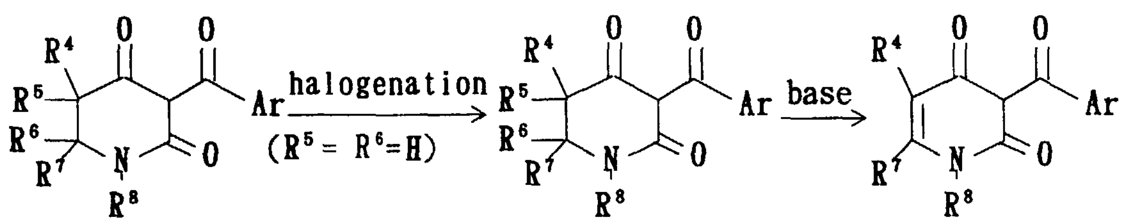


(1a) (4) (R5=halogen) (1b)

wherein $R^4$, $R^7$, $R^8$ and Ar are as defined above, and $R^5$ and $R^6$ are as defined in the reaction formula shown above.

A halogenated trione form (4) of compound (1a) can be prepared by allowing the compound (1a) prepared as described above to a reaction in a solvent with a halogenating agent, such as phenyltrimethylammonium bromide, in the presence of a base under an appropriate temperature in a range of from 0°C to a boiling point of the solvent used, and preferably from a room temperature to 50 °C for several to several douzen hours.

As an usable solvent in the reaction above, methylene chloride, benzene, ethyl acetate, THF, acetonitrile, DME and the like can be given as an example.

As examples for the solvent to be used, an alkali metal hydroxide, such as KOH and NaOH, an alkaline earth metal hydroxide, such as magnesium hydroxide and calcium hydroxide, tri($C_{1-6}$ alkyl)amine, such as triethylamine and diisopropylethylamine, an organic base, such as pyridine, sodium carbonate, sodium phosphate and the like can be given.

An objective compound (1b) can be prepared from the halogenated trione form (4) by dehydrohalogenation reaction in the presence of a base.

As examples for the base usable in the reaction, an alkali metal hydroxide, such as KOH and NaOH, an alkaline earth metal hydroxide, such as magnesium hydroxide and calcium hydroxide, tri($C_{1-6}$ alkyl)amine, such as triethylamine and diisopropylethylamine, an organic base, such as pyridine, sodium carbonate, sodium phosphate and the like can be given.

Manufacturing Method (iii)

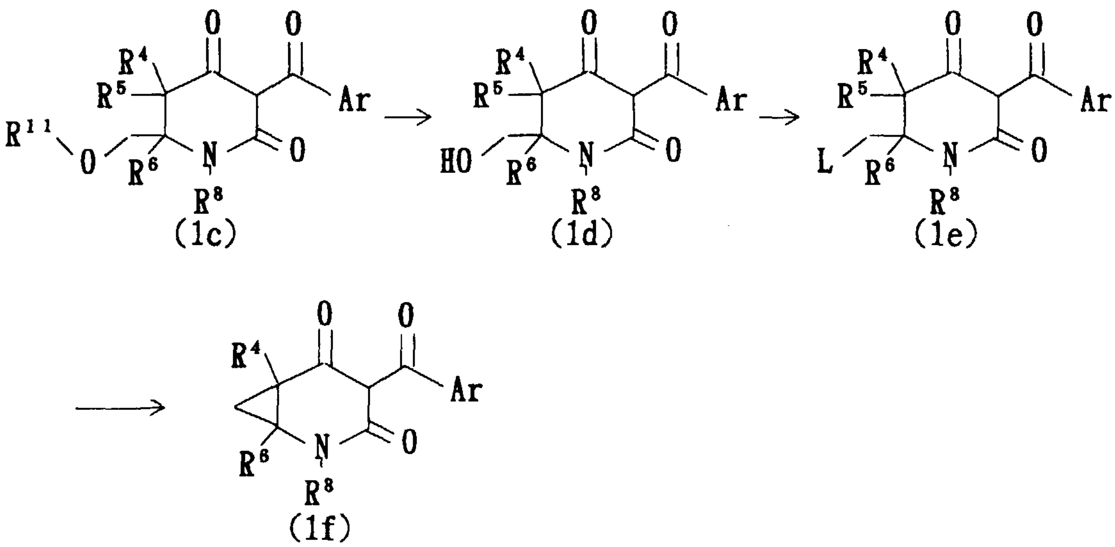

(1c) (1d) (1e) (1f)

In the reaction formula shown above, $R^4$, $R^5$, $R^6$, $R^8$ and Ar are as defined above, $R^{11}$ represents a lower branched or unbranched alkyl, aralkyl or acetyl, and L is an eliminating group, such as halogen, alkylsulfonate and arylsulfonate.

Compound (1d) can be prepared by subjecting compound (1c) to any of reactions with a hydrohalogenicacid, such as hydrochlonic acid or hydrobromic acid, trifluoroacetic acid or boron tribromide, hydrogenolysis or alkali hydrolysis and then subjecting the reacted-product to hydrolysis, if appropriate.

Also, the compound (1d) can be led to compound (1e) by subjecting it to halogenation, alkylsulfonation or arylsulfonation according to a customarily-known process.

Moreover, compound (1f) can be prepared by allowing the compound (1e) to a reaction in a solvent and in the presence of a base in an amount more than the equivalent mole under a temperature in a range of from -20 °C to a boiling point of the solvent used, and preferably from a room temperature to 100°C, during a period of from 30 min. to several dozen of hours.

As examples for the base to be used in the reaction described above, an alkali metal hydroxide, such as KOH and NaOH, an alkaline earth metal hydroxide, such as magnesium hydroxide and calcium hydroxide, tri ($C_{1-6}$ alkyl)amine, such as triethylamine and diisopropylethylamine, pyridine, 1,8-diazabicyclo[5,4,0]undecene-7 (DBU), t-BuOK, triton B, sodium carbonate, sodium phosphate and the like can be given.

As the solvent usable in the reaction, $H_2O$, alcohol, methylene chloride, benzene, toluene, ethyl acetate, DMF, THF, acetonitrile and the like can be given, and those solvents can be used either alone or in a form of mixture thereof.

ArCOQ, wherein Ar and Q are as defined above, and ArCOOH, wherein Ar is as defined above, are obtainable according to a process publicly-known.

A cyclic dione form represented by a general formula (2) can be prepared according to the process as shown below.

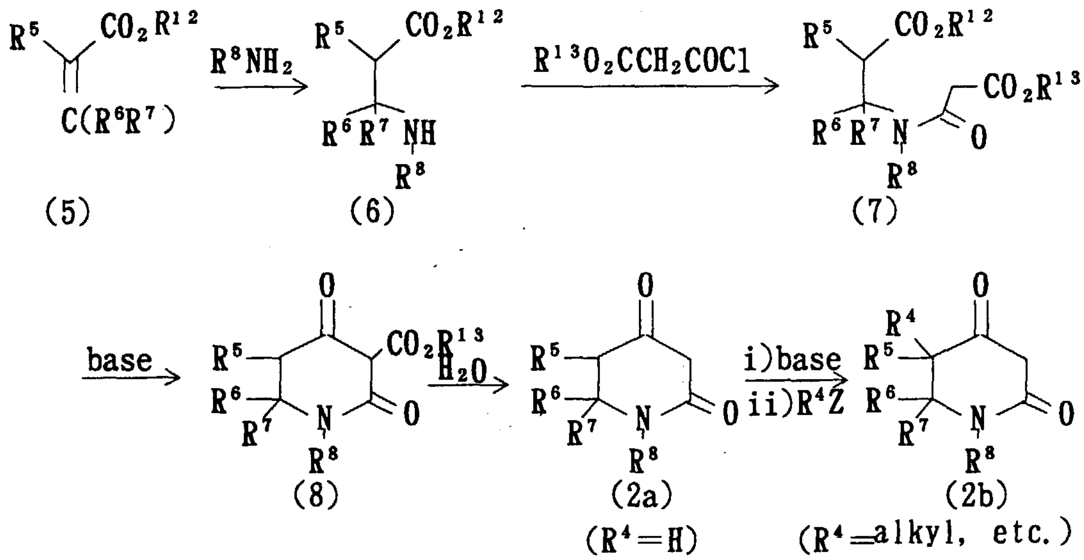


(5) (6) (7)

(8) (2a) ($R^4$=H) (2b) ($R^4$=alkyl, etc.)

wherein $R^5$, $R^6$, $R^7$ and $R^8$ are as defined above, $R^4$ is a group as shown in the reaction process above, $R^{12}$ and $R^{13}$ each represents independently a lower alkyl, and Z represents halogeno.

Compound (7) is obtainable by allowing compound (6), which is prepared from compound (5) and $R^8NH_2$ according to a process publicly-known, to a reaction with an acid chloride, $R^{13}O_2CCH_2COCl$, either in an amount of the equivalent mole or in an excess amount, in a solvent and in the presence of a base either in an amount of the equivalent mole or in an excess amount.

As examples for the base to be used in this reaction, an alkali metal hydroxide, such as KOH and NaOH, an alkali metal carbonate, such as sodium carbonate and potassium carbonate, an alkaline earth metal hydroxide, such as magnesium hydroxide and calcium hydroxide, tri($C_{1-6}$ alkyl)amine, such as triethylamine and diisopropylethylamine, an organic base, such as pyridine, sodium phosphate and the like can be given. As the solvent usable in this reaction, methylene chloride, chloroform, toluene, ethyl acetate, DMF, THF, DME, acetonitrile and the like can be given.

The reaction is carried out under a temperature in a range of from -10 °C to a boiling point of the solvent used.

Then, compound (8) is obtainable by allowing the compound (7) obtained as described above to a reaction in an organic solvent and in the presence of any of an alkali metal alcolate, such as sodium ethylate, a metal hydride, such as sodium hydride, and the like, in an amount of from the equivalent mole to 3 moles, respectively, under a temperature in a range of from 0°C to a boiling point of the solvent used, and by subsequently subjecting the reacted-product to neutrization process by using an inorganic acid, such as hydrochloric acid.

As examples for the solvent used in the reaction above, an alcohol, such as ethanol and methanol, an ether, such as THF and DME, a hydrocarbon, such as toluene and xylene, DMF, and the like can be given.

Then, compound (2a) is obtainable by heating the compound (8) obtained in a solvent and in the presence of $H_2O$.

As the solvent used in the above reaction, methylene chloride, chloroform, toluene, ethyl acetate, DMF, THF, dioxane, DME, acetonitrile and the like can be used.

Further, compound (2b) is obtainable by allowing the compound (2a) to a reaction with an electrophilic reagent, $R^4Z$, in the presence of a base under a temperature in a range of from -78°C to a boiling point of the solvent used.

As examples for the solvent described above, ether, THF, benzene, toluene, hexane and the like can be given. Whereas, as the base described above, an alkyl lithium, lithium diisopropylamide and the like can be used.

The acrylate derivatives to be used as a raw material can be prepared according to a publicly-known process, and the representative examples are given in the following;

a) 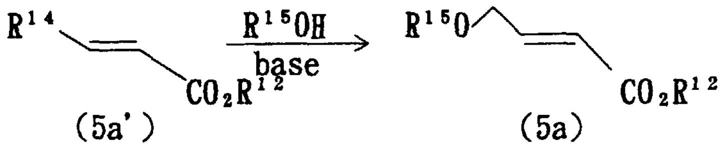


(5a') (5a)

b) 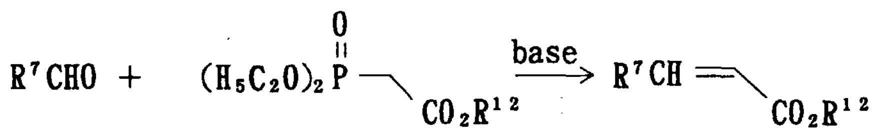


(5b)

wherein $R^7$ and $R^{12}$ are as defined above, $R^{14}$ represents an eliminating group, such as chlorine and bromine, and $R^{15}$ represents alkyl, alkenyl, haloalkyl, or the like.

An acrylate derivative (5a) is obtainable by allowing a halogenated acrylate derivative (5a') to a reaction in an inactive solvent with an alcohol, $R^{15}OH$, in the presence of a base under a temperature in a range of from -10°C to a boiling point of the solvent used. In addition, the acrylate derivative (5b) is also obtainable from an aldehyde derivative by employing Wittig reaction.

An aldehyde form (8) and a carboxylate form (9), which are important intermediates for preparing the compounds of the present invention can be prepared according to the process shown in the following;

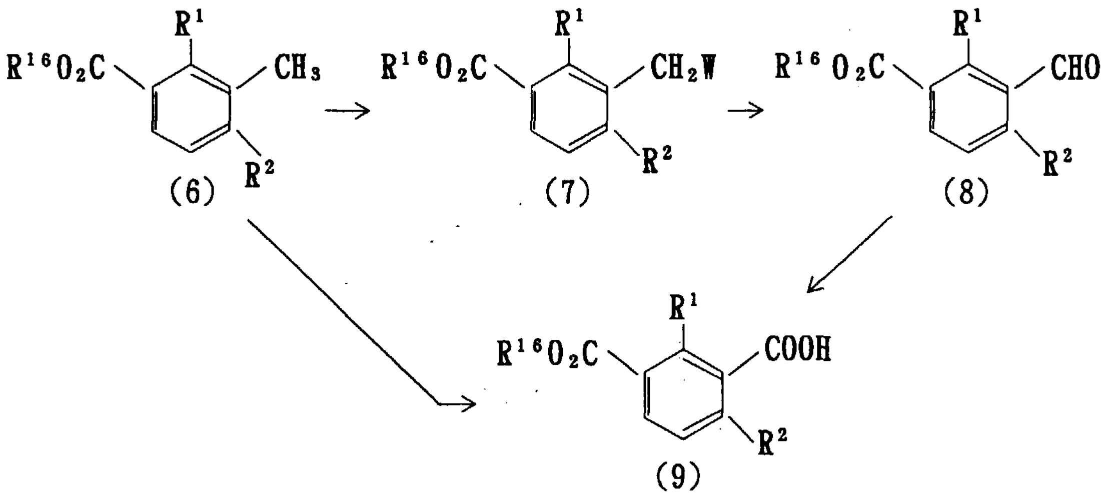

(6) (7) (8)

(9)

wherein $R^1$ and $R^2$ are as defined above, $R^{16}$ represents hydrogen or a lower alkyl, and W represents halogeno.

The aldehyde form (8) is obtainable by firstly preparing a benzylhalide derivative (7) from a toluene derivative (6) according to a publicly-known process, for example, which prepares said benzyl halide derivative by allowing the toluene derivative (6) to a reaction with halogen, such as chlorine and bromine, or a halogenating agent, such as N-bromosuccineimide (NBS) and N-chlorosuccineimide (NCS), in the presence of a radical reaction initiator, such as benzoyl peroxide, and then by transforming the benzyl halide derivative (7) to the aldehyde form (8) according to a process published in J. Am. Chem. Soc., 71, 1767 (1949). Namely, the aldehyde form (8) can be prepared by allowing the obtained benzyl halide derivative (7) to a reaction in an alcohol, such as methanol and ethanol, with an alkali metal salt of a nitroalkane compound, such as 2-nitropropane, under a temperature in a range of from 0 °C to a boiling point of the solvent used.

Whereas, a carboxylic acid form (9) is obtainable by allowing the toluene derivative (6) to an oxidation reaction with potassium permanganate, etc. or from the aldehyde form (8) by allowing the aldehyde form (8) to an oxidation reaction according to a publicly-known process, for example, with any of a Jones reagent, chromic acid and potassium permanganate, or the like.

Furthermore, the following intermediates are also obtainable by using the aldehyde form (8) and the carboxylic

acid form (9) as shown below;

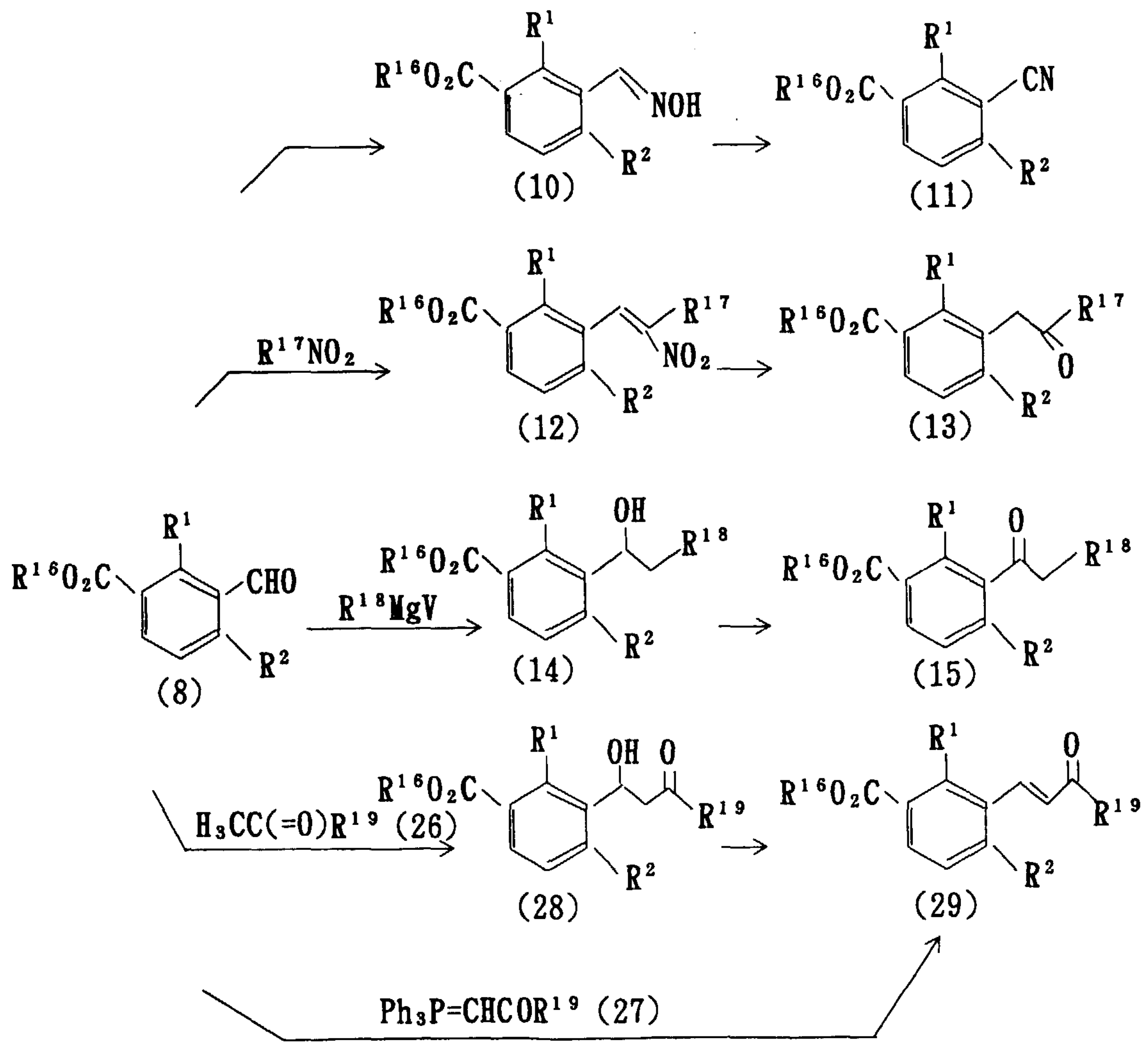


wherein $R^1$, $R^2$, and $R^{16}$ are as defined above, $R^{17}$ and $R^{18}$ each represents hydrogen or a lower alkyl, V represents halogeno, and $R^{19}$ represents a lower alkyl.

An aldoxime form (10) is obtainable by allowing the aldehyde form (8) to a reaction with either hydroxylamine chloride or hydroxylamine sulfate in the presence of a base. Further, a cyano form (11) is obtainable by allowing the aldoxime form (10) to a reaction with a dehydrating agent, such as acetic anhydride, diphosphorus pentaoxide and thionyl chloride.

Whereas, a ketone form (13) is obtainable by firstly preparing a nitroolefin form (12), for example, by employing Knoevenagel condensation reaction described in Organic Reactions, Vol. 15, page 254, then reducing the nitroolefin form (12) with an activated iron-water system, lithium aluminium hydride or the like, and subsequently subjecting the reduced nitroolefin form (12) to hydrolysis.

An acyl form (15) is obtainable by firstly preparing an alcohol form (14) by allowing the aldehyde form (8) to a reaction with a Grignard reagent, and subsequently subjecting the alcohol form (14) to oxidation with an oxidizing agent, such as activated manganese dioxide and chromic acid.

A vinyl ketone form (29) is obtainable by firstly preparing an aldol form (28), which is prepared by allowing the aldehyde form (8) to a reaction in water for 1-50 hours with methyl ketone (26) according to a publicly-known process in the presence of a catalyst and under a temperature in a range of from 0 to 50 °C, and subsequently subjecting the obtained aldol form (28) to dehydration in an appropriate solvent and in the presence of a catalyst. As the catalyst to be used in the reaction to prepare said aldol form (28), a metal hydroxide, such as sodium hydroxide and barium hydroxide, and an organic base, such as piperidine and pyridine, can be used.

Whereas, as the catalyst to be used in the dehydration reaction described above, an acid, such as concentrated sulfuric acid and p-toluenesulfonic acid, can be used. Also, as the solvent usable in the dehydration reaction, hydrocarbons, such as benzene and toluene, and halogenated hydrocarbons, such as methylene chloride and chloroform, can be given as an example.

Whereas, the vinyl ketone from (29) is also obtainable by allowing the aldehyde form (8) to a reaction with phosphorane (27) for 10 min. to 5 hours in an appropriate solvent under a temperature in a range of from a room temperature to a boiling point of the solvent used.

An amide form (17), a hydrazide form (18) and a β-diketone form (20) are obtainable according to the processes shown hereinbelow, respectively;

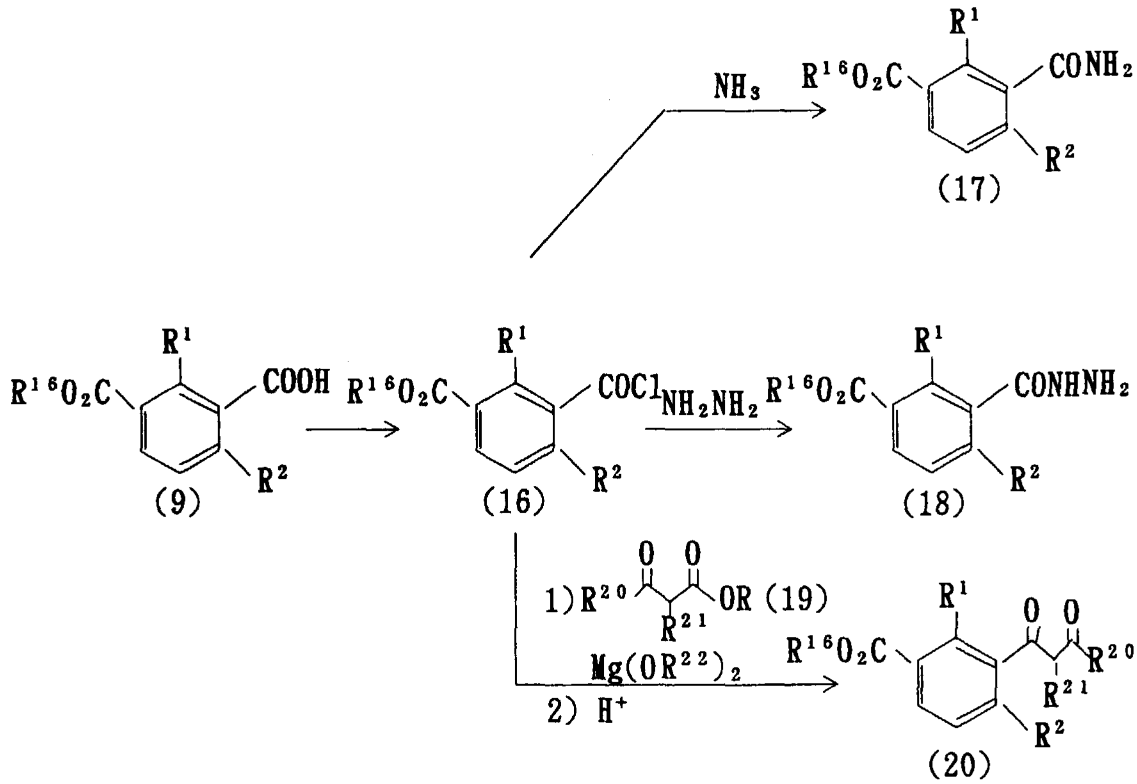


(17) (9) (16) (18) (20)

wherein $R^1$, $R^2$ and $R^{16}$ are as defined above, and R, $R^{20}$, $R^{21}$ and $R^{22}$ each represents independently a lower alkyl.

At first, an intermediate, a carbonyl chloride form (16) is prepared by allowing a carboxylic acid form (9) to a reaction with a chlorinating agent, such as phosgene, thionyl chloride and oxalyl chloride, in an inactive solvent, such as hydrocarbons including benzene and toluene, and halogenated hydrocarbons including methylene chloride and chloroform.

Subsequently, according to a publicly-known process, the amide form (17) and the hydrazid form (18) can be obtained from the carbonyl chloride form (16). Whereas, the β-diketone form (20) is obtainable by allowing a magnesium salt of the β-keto ester form (19), which is prepared by subjecting the β-keto ester form (19) to a reaction with magnesium alcolate, to a reaction with the carbonyl chloride form (16) according to a publicly-known process.

Now, a process for preparing heterocyclic intermediates is described hereinbelow.

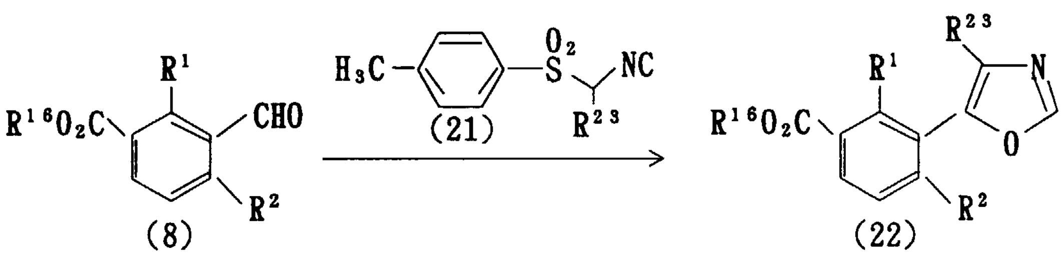

(8) (21) (22)

In the reaction formula shown above, $R^1$, $R^2$ and $R^{16}$ are as defined above, and $R^{23}$ corresponds to $R^9$ or $R^{10}$ as defined above.

An oxazole form represented by a general formula (22) is obtainable by allowing an aldehyde form (8) to a reaction with an isonitrile form (21) in an adequate solvent for 1 to 30 hours in the presence of a base under a temperature in a range of from a room temperature to a boiling point of the solvent used according to the process described in a published reference, for example, Tetrahedron Letters., (23) 2369 (1972) or the else.

As examples for the base used in the reaction described above, a carbonate, such as sodium hydrogencarbonate and potassium carbonate, an alkali metal hydroxide, such as sodium hydroxide and potassium hydroxide, a metal alcolate, such as sodium methylate and sodium ethylate, and an organic base, such as triethylamine and DBU, can be given. And, as examples for the solvent used in said reaction, an alcohol, such as methanol, ethanol and isopropanol, a hydrocarbon, such as benzene and toluene, a halogenated hydrocarbon, methylene chloride and chloroform, an ether, such as THF and dioxane, a nitrile, such as acetonitrile, and DMF can be given.

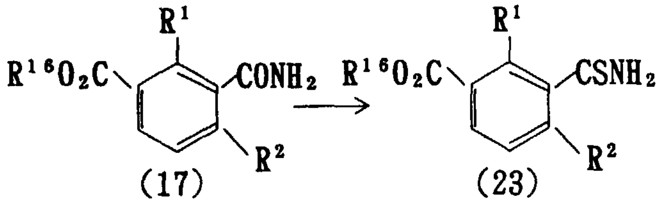

(17) (23)

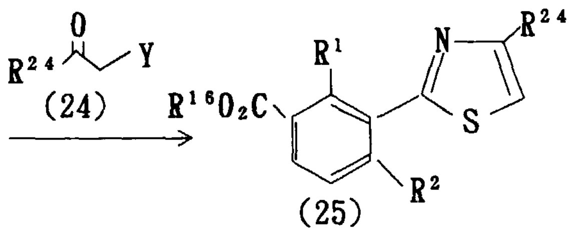

(24) (25)

In the reaction formula shown above, $R^1$, $R^2$ and $R^{16}$ are defined above, and $R^{24}$ corresponds to $R^9$ or $R^{10}$ as defined above, and Y represents a halogen atom.

A thiazole-type derivative represented by a general formula (25) is obtainable from an amide form derivative (17) via a thioamide form derivative (23). In this process, the thioamide form (23) is prepared by allowing the amide form (17) to a reaction with either phosphorus pentasulfide or Lawson reagent either in a solvent or without solvent under a temperature in a range of from a room temperature to a boiling point of the solvent used. As examples for the solvent used in the reaction, a hydrocarbon, such as benzene and toluene, and an ether, such as dioxane, can be given.

Then, the thiazole form (25) can be obtained by allowing the thioamide form (23) obtained to a reaction with an $\alpha$-haloketone form (24) in an adequate solvent for 1 to 30 hours under a temperature in a range of from a room temperature to a boiling point of the solvent used either in the presence of a base or without base.

As examples for the base used in the reaction, a carbonate, such as sodium hydrogen carbonate and potassium carbonate, an alkali metal hydroxide, such as sodium hydroxide and potassium hydroxide, a metal alcoholate, such as sodium methylate and sodium ethylate, and an organic base, such as triethylamine and DBU, can be given.

Further, as example for the solvent used in the reaction, an alcohol, such as methanol, ethanol and isopropanol, a hydrocarbon, such as benzene and toluene, an halogenated hydrocarbon, such as methylene chloride and chloroform, a ketone, such as acetone and methyl ethyl ketone, an ester, such as methyl acetate and ethyl acetate, an ether, such as THF and dioxane, a nitrile, such as acetonitrile, and DMF can be given.

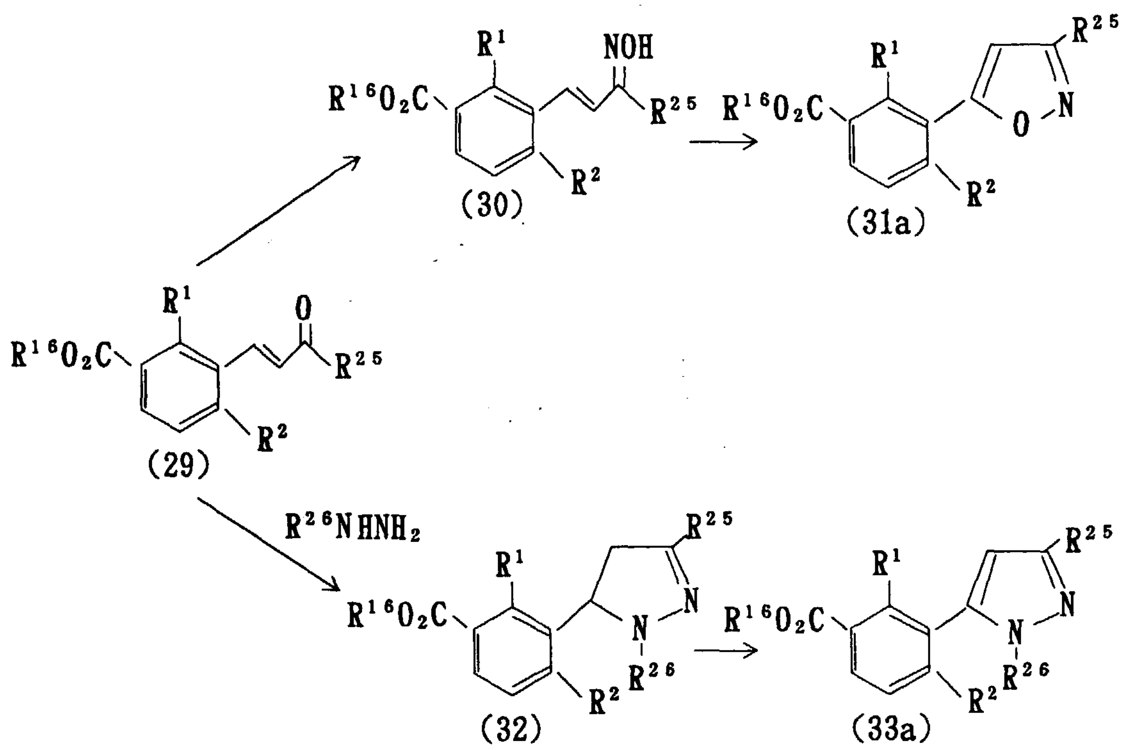


In the reaction formula shown above, $R^1$, $R^2$ and $R^{16}$ are as defined above, and $R^{25}$ and $R^{26}$ each corresponds to $R^9$ or $R^{10}$ as defined above.

An isoxazole-type derivative (31a) is obtainable by firstly preparing an oxime form (30) by allowing a vinyl ketone form (29) to a reaction with hydroxylamine in an adequate solvent for 0.5 to 5 hours under a temperature of from 0°C to a boiling point of the solvent used and subsequently subjecting the oxime form to a ring closure reaction and an oxidation reaction. In this reaction, the reaction with hydroxylamine can be carried out by using hydroxylamine sulfate or hydroxylamine hydrochloride, namely using the salt without adjusting the pH to neutral, but the reaction can be done after the neutralization by using an adequate base. As examples for the base used for the said neutralization, a carbonate, such as sodium hydrogencarbonate and potassium hydrogencarbonate, an alkali metal hydroxide, such as sodium hydroxide and potassium hydroxide, a carboxylate, such as sodium acetate, a metal alcoholate, such as sodium methylate and sodium ethylate, and an organic base, such as triethylamine and pyridine, can be given.

And, as examples for the solvent used in the reaction described hereinabove, an alcohol, such as methanol, ethanol and isopropanol, a hydrocarbon, such as benzene and toluene, a halogenated hydrocarbon, such as methylene chloride and chloroform, an ether, such as THF and dioxane, a nitrile, such as acetonitrile, DMF, pyridine, acetic acid and $H_2O$, and mixtures of 2 or more solvents described above. For the ring closure reaction and the oxidation reaction, iodine-potassium iodide, N-bromosuccinimide or palladium catalyst or the like are used, which can be prepared according to the process described in J. Am. Chem. Soc., 94, 9128 (1972); J. Heterocycle. Chem. , 14, 1289 (1977); and Tetrahedron Lett. 1977, 5075, respectively.

A pyrazole form (33a) is also obtainable from a vinyl ketone form (29) via two steps. Namely, a vinyl ketone form (29) is allowed to a reaction with a substituted hydrazine in an appropriate solvent for 0.5 to 5 hours under a temperature in a range of 0 °C to a boiling point of the solvent used to obtain a dihydropyrazole form (32).

As examples for the solvent used in this reaction, an alcohol, such as methanol, ethanol and isopropanol, a hydrocarbon, such as benzene and toluene, a halogenated hydrocarbon, such as methylene chloride and chloroform, an ether, such as THF and dioxane, a nitrile, such as acetonitrile, DMF, pyridine, acetic acid and $H_2O$, and mixtures of 2 or more solvents described above, can be given.

The oxidation reaction is proceeded by allowing the dihydropyrazole form (32) to a reaction with an oxidizing agent selected from activated manganese dioxide, 2,3-dichloro-5,6-dicyanobenzoquinone (DDQ), nickel peroxide, NBS or the like in an appropriate solvent under a temperature in a range of from a room temperature to a boiling point of the solvent used. As the solvent used in this reaction, a hydrocarbon, such as benzene and toluene, and a halogenated hydrocarbon, such as chloroform and carbon tetrachloride, can be given, for example.

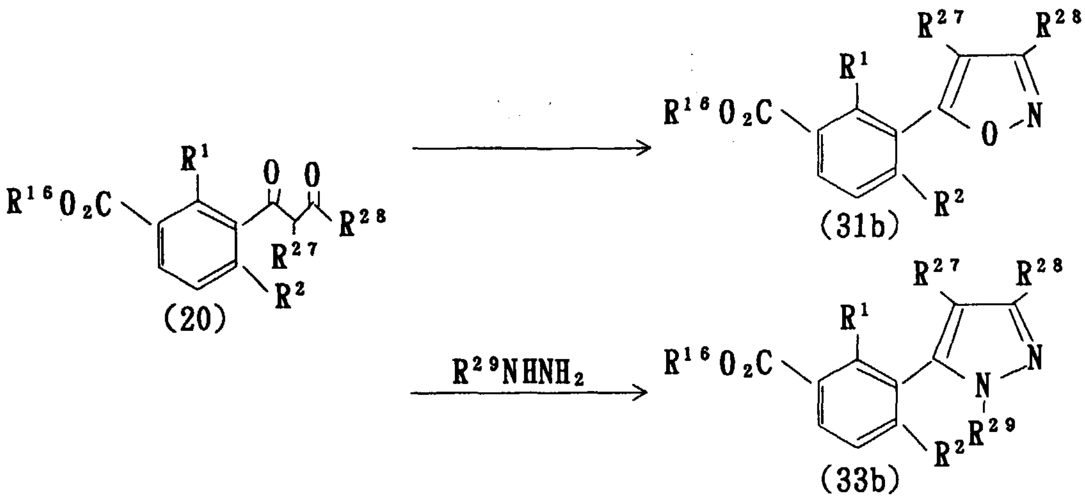


(20) (31b) (33b)

In the reaction formula shown above, $R^1$, $R^2$ and $R^{16}$ are as defined above, and $R^{27}$, $R^{28}$ and $R^{29}$ each corresponds to $R^9$ or $R^{10}$ as defined above.

Further, an isoxazole form represented by a general formula (31b) and a pyrazole form represented by a general formula (33b) are also obtainable by allowing a diketone form (20) to a reaction with hydroxylamine and a substituted pyrazole, respectively. The reaction described hereinabove is proceeded in an appropriate solvent under a temperature in a range of from 0 °C to a boiling point of the solvent used. In this reaction, an acid, such as sulfuric acid and p-toluenesulfonic acid can be used as a catalyst. And, as examples for the solvent usable in the reaction, an alcohol, such as methanol, ethanol and isopropanol, a hydrocarbon, such as benzene and toluene, a halogenated hydrocarbon, such as methylene chloride and chloroform, an ether, such as THF and dioxane, a nitrile, such as acetonitrile, DMF, pyridine, acetic acid and $H_2O$, and mixtures of 2 or more solvents described above, can be given.

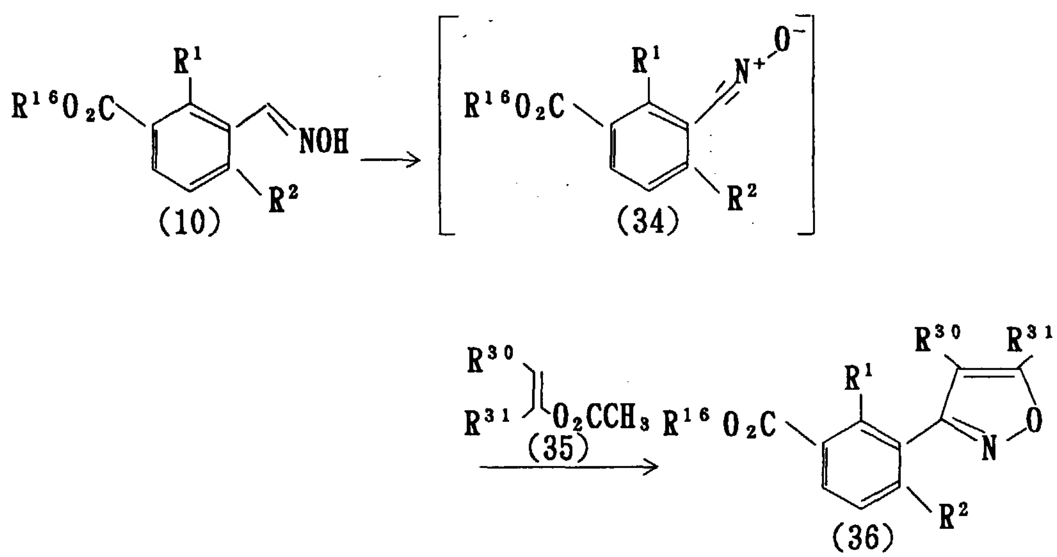

(10) (34) (35) (36)

In the reaction formula shown above, $R^1$, $R^2$ and $R^{16}$ are as defined above, and $R^{30}$ and $R^{31}$ each corresponds to $R^9$ or $R^{10}$ as defined above.

An isoxazole form represented by a general formula (36) is obtainable by firstly preparing a nitrile oxide form (34) by allowing an aldoxime form (10) to a reaction with a halogenating agent, such as chlorine, bromine, NCS and NBS, in a solvent selected from hydrocarbons, such as benzene and toluene, halogenated hydrocarbons, such as

methylene chloride and chloroform, ethers, such as THF and dioxane, nitriles, such as acetonitrile and DMF under a temperature in a range from -10 °C to 50°C and then allowing the reacted-aldoxime form to a reaction with a base selected from organic bases, such as triethylamine, and carbonates, such as sodium hydrogencarbonate and potassium carbonate, and subsequently allowing the obtained nitrile oxide form to a reaction with a vinyl acetate form (35) under a temperature in a range of from a room temperature to a boiling point of the solvent used. Whereas, an isoxazole form (36) can be also prepared by allowing the halogenated aldoxime form to a reaction with a base selected from ones as described above in the presence of a vinyl acetate form (35).

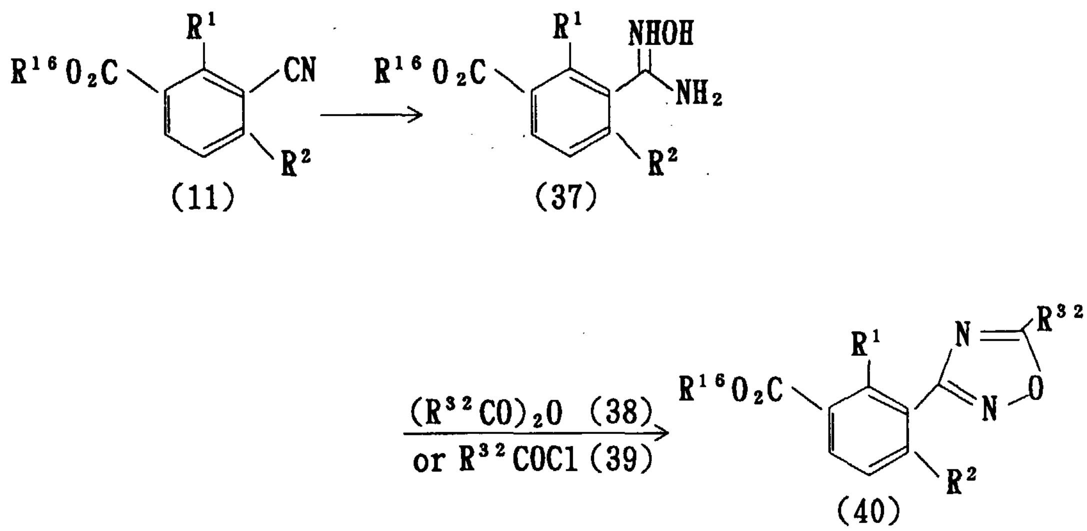


(11) (37) (40)

In the reaction formula shown above, $R^1$, $R^2$ and $R^{16}$ are as defined above, and $R^{32}$ corresponds to $R^9$ as defined above.

An oxadiazole form (40) can be prepared via an amide oxime form (37). The amide oxime form (37) is obtainable by allowing a nitrile form (11) to a reaction with hydroxylamine in an appropriate solvent under a temperature in a range of from a room temperature to a boiling point of the solvent used. Whereas, hydroxylamine is used in neutralized state by neutralizing of hydroxylamine sulfate or hydroxylamine hydrochloride with an adequate base selected from carbonates, such as sodium hydrogencarbonate and potassium carbonate, alkali metal hydroxides, such as sodium hydroxide and potassium hydroxide, carboxylates, such as sodium acetate, metal alcoholates, such as sodium methylate and sodium ethylate, and organic bases, such as triethylamine and pyridine. As examples for the solvent used for this reaction, an alcohol, such as methanol, ethanol and isopropanol, a hydrocarbon, such as benzene and toluene, a halogenated hydrocarbon, such as methylene chloride and chloroform, an ether, such as THF and dioxane, a nitrile, such as acetonitrile, DMF, pyridine, acetic acid and $H_2O$, and mixtures of 2 or more solvents described above can be given.

Then, an oxadiazole form (40) is obtainable by allowing the obtained amidoxime form (37) to a reaction with an acid anhydride (38) or an acid chloride (39) in an appropriate solvent for 1 to 30 hours in the presence of an adequate base under a temperature in a range of from -15 °C to a boiling point of the solvent used.

As examples for the base usable in this reaction, a carbonate, such as sodium hydrogencarbonate and potassium carbonate, an alkali metal hydroxide, such as sodium hydroxide and potassium hydroxide, and an organic base, such as triethylamine, pyridine and DBU, can be given.

Whereas, as examples for the solvent usable for this reaction, an hydrocarbon, such as benzene and toluene, a halogenated hydrocarbon, such as methylene chloride and chloroform, an ether, such as THF and dioxane, a nitrile, such as acetonitrile, DMF, pyridine and the like can be given.

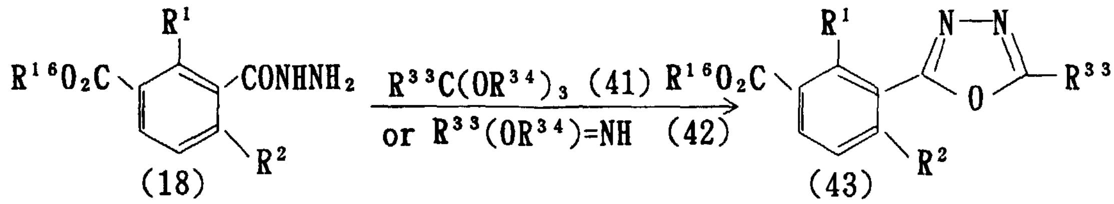


(18) (43)

In the reaction formula shown above, $R^1$, $R^2$ and $R^{16}$ are as defined above, $R^{33}$ corresponds to $R^9$ as defined above, and $R^{34}$ represents a lower alkyl.

An oxadiazole form (43) is obtained by allowing a hydrazid form (18) to a reaction with either of an ortho-ester form (41) or an imidate form (42) in an appropriate solvent for 1 to 30 hours under a temperature in a range of from -15°C to a boiling point of the solvent used. As the solvent used for this reaction, a hydrocarbon, such as benzene and toluene, a halogenated hydrocarbon, such as methylene chloride and chloroform, an ether, such as THF and dioxana, a nitrile, such as acetonitrile, DMF, pyridine and the like can be given as an example.

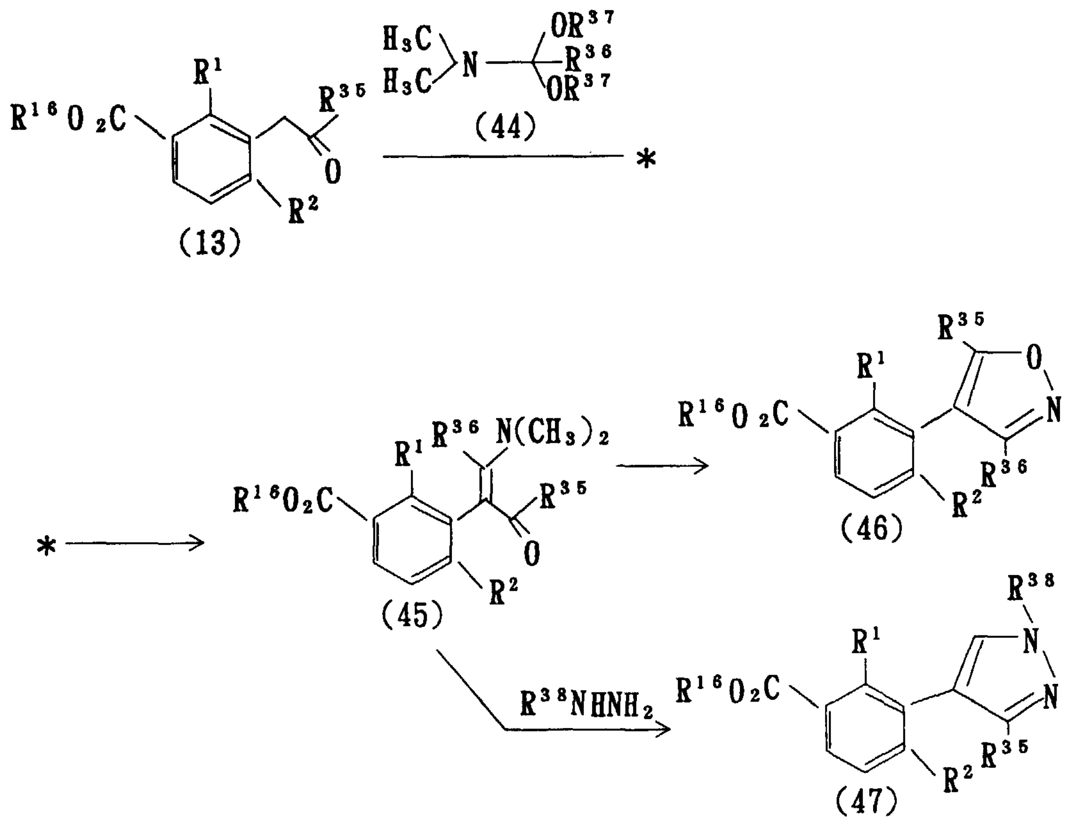


(13) (45) (46) (47)

In the reaction formula shown above, $R^1$, $R^2$ and $R^{16}$ are as defined above, and $R^{35}$, $R^{36}$ and $R^{38}$ each corresponds to $R^9$ or $R^{10}$ as defined above, and $R^{37}$ represents a lower alkyl.

An isoxazole form represented by a general formula (46) is obtainable from an ketone form (13) via a dimethylaminomethylidyne form (45). Namely, the isoxazole form is obtainable by allowing the ketone form (13) to a reaction with a dimethylamideacetal form (44) either in an appropriate solvent or without solvent under a temperature in a range of from a room temperature to either 200 °C or a boiling point of the solvent used. As the solvent usable in this reaction, a hydrocarbon, such as toluene and xylene, can be given as an example.

Then, the methylaminomethylidyne form (45) obtained is allowed to a reaction with hydroxylamine to prepare the isoxazole form (46) according to the same process described above for preparing an isoxazole form (31b).

Whereas, a pyrazole form represented by a general formula (47) is also prepared by allowing the methylaminomethylidyne form (45) to a reaction with a substituted hydrazine according to the same process described above for preparing a pyrazole form (33b).

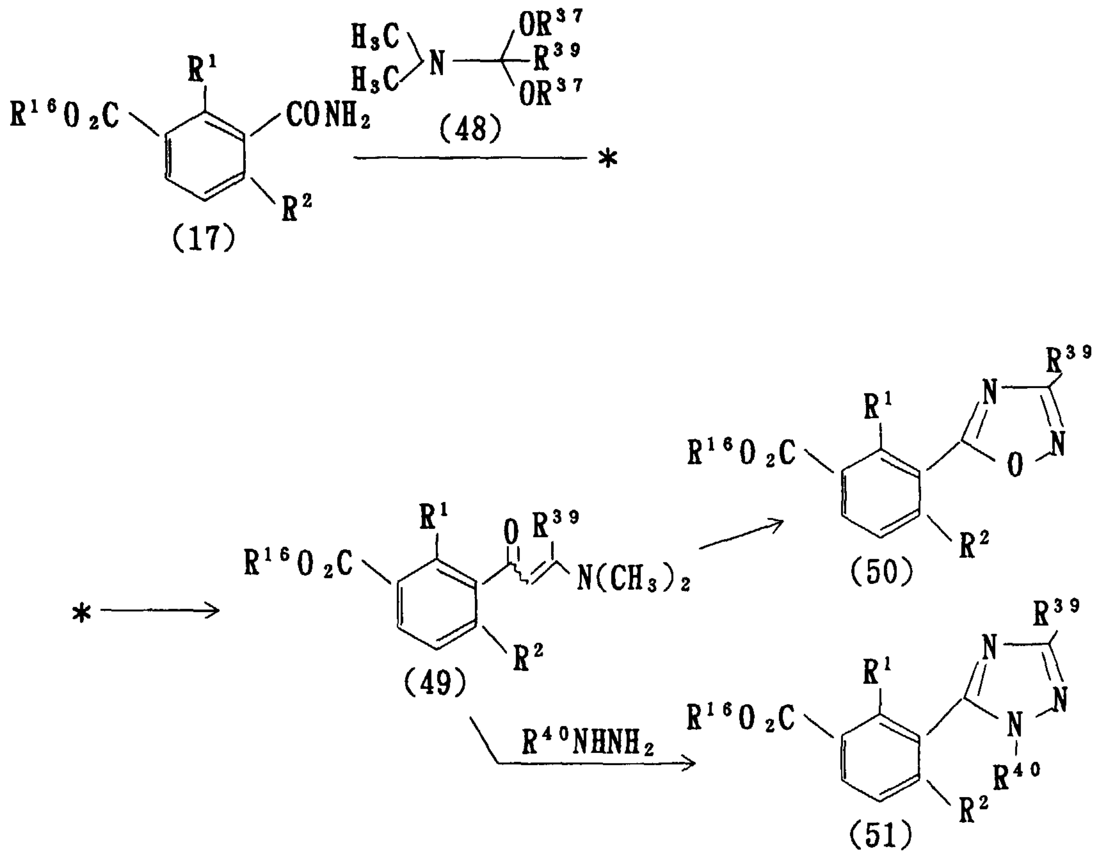


(17) (48) (49) (50) (51)

In the reaction formula shown above, $R^1$, $R^2$, $R^{16}$ and $R^{37}$ are as defined above, and $R^{39}$ and $R^{40}$ each corresponds to $R^9$ or $R^{10}$ as defined above.

An oxadiazole form represented by a general formula (50) is prepared from an amide form (17) via an amidine form (49). Namely, the amidine form (49) is obtainable by allowing the amide form (17) to a reaction with a dimethylamideacetal form (48) either without solvent or in an appropriate solvent under a temperature in a range of from 0°C to either 200°C or a boiling point of the solvent used. As examples for the solvent used for this reaction, a hydrocarbon, such as toluene and xylene, can be given. The amidine form (49) obtained is then allowed to a reaction with hydroxylamine to obtain the oxadiazole form (50) according to the same process as described above for preparing the isoxazole form (31b). Further, a triazole form represented by a general formula (51) is also prepared by allowing the amidine form (49) to a reaction with a substituted hydrazine according to the same process as described above for preparing the pyrazole for (33b).

a)

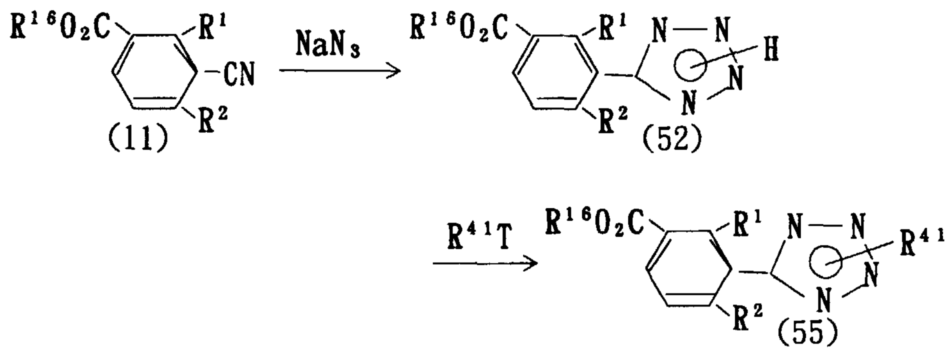


(11) (52) (55)

b)

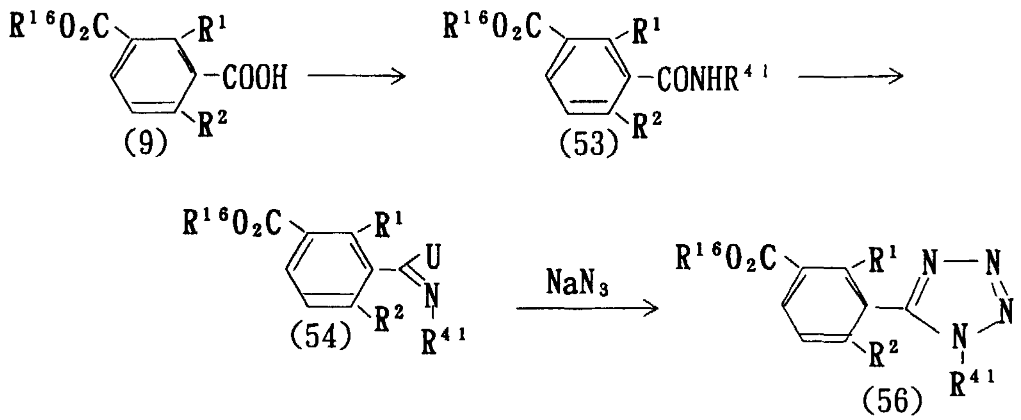


(9) (53) (54) (56)

In the reaction formula shown above, $R^1$, $R^2$ and $R^{16}$ are as defined above, $R^{41}$ corresponds to $R^9$ as defined above, T represents a halogen atom or sulfonate, and U represents a halogen atom or a lower alkoxy.

Whereas, tetrazole derivatives are obtainable according to the processes described in the following manuscripts; P. K. Kadaba, Synthesis, 1973 71; M. F. Ansell, Rodd's Chemistry of Carbon Compounds, IVD, 211-237, Elsevier Science Publishers (1986); and R. N. Butler, Advances in Heterocyclic Chem., 21 323-436, Academic Press (1977).

According to these references, compound (52) is obtainable by allowing a cyano form (11) to a reaction with either of an azide, such as sodium azide, lithium azide and ammonium azide, or sodium azide and ammonium chloride in a solvent selected from DMF, N,N-dimethylacetoamide (DMA), dimethylsulfoxide (DMSO), acetonitrile, etc. for 1 to 48 hours under a temperature in a range of from a room temperature to a boiling point of the solvent used.

Then, a tetrazole form (55) is obtainable by subjecting the compound (52) to normal alkylation process. For example of the alkylation process, a process to allow the compound (52) to a reaction with an alkyl halide or an alkyl sulfonate in a solvent selected from ethers, alcohols, acetonitrile, DMF, DMSO and the like and in the presence of any of an alkali metal hydroxide, an alkali metal carbonate, an organic amine and a metal halide under a temperature in a range of from -10 °C to a boiling point of the solvent used can be given.

Alternatively, the tetrazole form (56) is also obtainable by firstly preparing an imidate form (54) from a N-alkylamide form (53), which is obtained according to a customarily-known process from a carboxylic acid form (9), according to a customarily-known process and subsequently allowing the imidate form (54) to a reaction with any of the azides as described above in a solvent selected from acetone, $H_2O$, DMF, DMA, DMSO and the like under a temperature of from -10 °C to 50°C.

Benzoates represented by a formula (I-1) shown below are obtainable by firstly preparing a 4-SR' form represented by a formula (I-3) shown below by allowing a 4-Cl form represented by a formula (I-2) shown below to a reaction with a thiol represented by a formula, R'SH, in the presence of a base, and subsequently oxidizing the 4-SR' form;

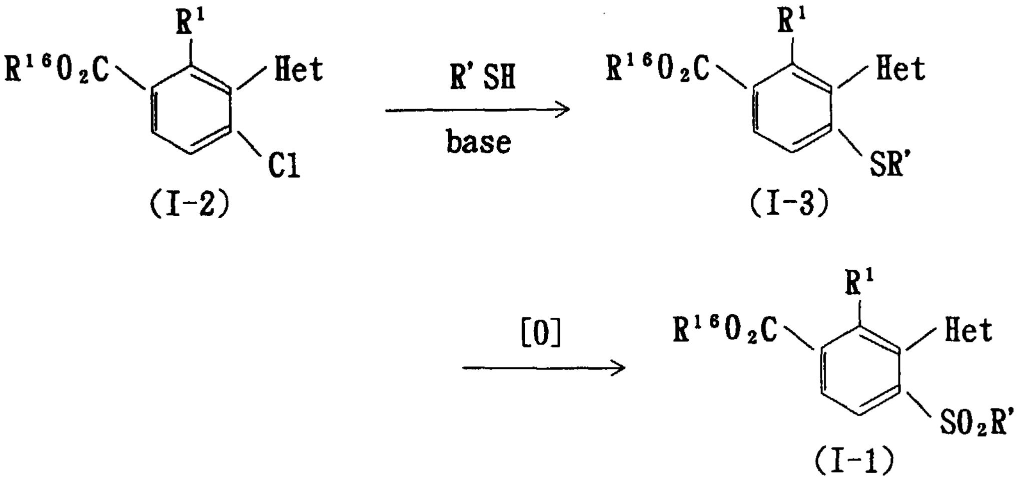


wherein $R^1$, $R^{16}$ and Het are as defined above, and R' represents a $C_{1-6}$ alkyl.

As examples for the base usable for this reaction, an alkali metal hydroxide, such as sodium hydroxide and potassium hydroxide, a metal alkoxide, such as sodium methoxide and sodium ethoxide, a carbonate, such as sodium carbonate and potassium carbonate, a hydride, such as sodium hydride, and an organic base, such as triethylamine, diisopropylethylamine, DBU and pyridine, can be given. Also, as examples for said solvent, an alcohol, such as methanol and ethanol, an ether, such as THF and DME, an amide, such as DMF and DMA, a hydrocarbon, such as benzene, toluene and xylene, DMSO, acetonitrile and the like can be given.

The following oxidation reaction is proceeded in an inactive solvent selected from $H_2O$, organic acids, such as acetic acid, and halogenated hydrocarbons, such as methylene chloride, chloroform and carbon tetrachloride, by using an oxidizing agent, such as a peroxy acid including hydrogen peroxide, peracetic acid, perbenzoic acid and m-chloroperbenzoic acid, and a hypochlorous acid including sodium hypochloride and potassium hypochloride.

The reaction proceeds smoothly in a temperature range of from a room temperature to a boiling point of the solvent used.

The inventive compounds (1) and their starting compounds (2) and (8) may contain their optical isomers and various tautomers, as shown in the following. All of these isomers and taumers shall fall within the scope of the compounds according to the present invention.

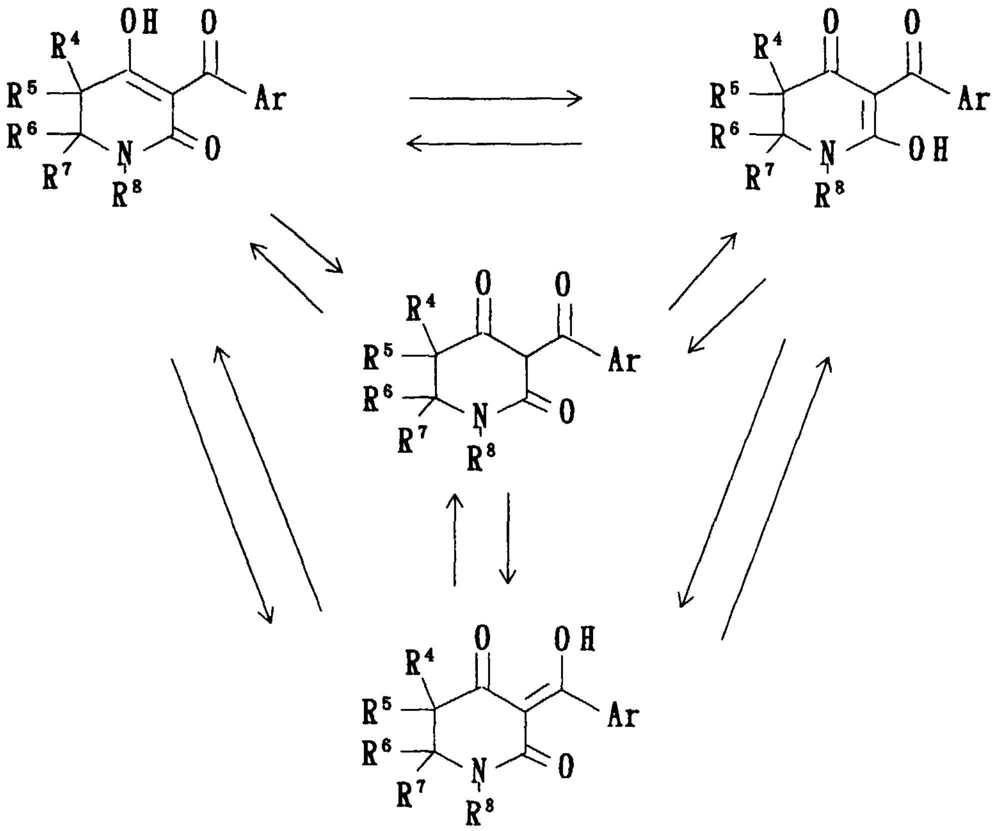

When compound (1) contains a free hydroxyl group, it is possible to prepare from the compound (1) its salts, particularly the salts acceptable for agricultural and horticultural use, and its derivatives, such as enamines and the analogs thereof, acylates, sulfonates, carbamates, ethers, thioethers, sulfoxides and sulfones. As examples for the salts acceptable for agricultural and horticultural use, sodium, potassium, calcium and ammonium salts can be given.

As the ammonium salts, salts with $N^+$ RaRbRcRd, wherein Ra, Rb, Rc and Rd each independently represents hydrogen or a $C_{1-10}$ alkyl optionally-substituted with hydroxy or the like, is given as an example. When any of Ra, Rb, Rc and Rd is an optionally-substituted alkyl, it is preferable that the alkyl group contains 1-4 carbon atoms.

As examples for the preferable enamines and the analogs thereof, the ones of which OH-group part being converted to a group represented by a formula, -NReRf, wherein Re and Rf each independently represents hydrogen or $C_{1-6}$ optionally-substituted alkyl or aryl, such as phenyl, halogeno and S(O)gRh, wherein Rh represents, for example, $C_{1-6}$ optionally-substituted alkyl or aryl, such as phenyl and g is 0, 1 or 2, respectively, can be given.

Whereas, as examples for the preferable derivatives, such as acylates, ethers and carbamate derivatives, compounds of which OH-group part being converted to a groups respectively represented by formulas, - OCORi, -ORj and -OCONRkRl, wherein Ri and Rj each corresponds to Rh as defined above and Rk and Rl each corresponds to Re as defined above, can be given.

These derivatives as described above can be prepared according to customarily-known processes.

The objective compounds according to the present invention can be obtained after normal post-reaction procedure following to the respective reaction.

The determination of the structures of the inventive compounds were made based on IR, NMR, MS, etc.

The compounds according to the present invention provide high herbicidal activity against weeds grown in the fields of upland crops by either way of soil application or foliar application. For example, the compounds according to the present invention show to have an excellent herbicidal activity against various weeds in upland crop fields, such as giant foxtail, common cocklebur, barnyardglass, velvetleaf, etc., and show to be selectively safe to a certain crops, such as maize.

Further, among the compounds of the present invention, such compounds that have a growth-regulating activity, such as growth retardants, against useful plants including agricultural crops, ornamental plants and fruit trees are also included as well.

Moreover, the compounds according to the present invention can be applied for weed control in orchards, lawns, railways side, vacant lands, etc.

The herbicide according to the present invention comprises one or more of the compounds according to the present invention as an active ingredient. At practical application of the inventive compounds, such compound can be applied alone without combining with other elements. Alternatively, such inventive compounds can be also prepared into any formulation types having been normally employed for plant protection chemicals, such as wet table powders, granules, dusts, emulsifiable concentrates, water soluble powders, suspensions and flowable formulations. As an additive or a carrier to be used for such formulations, vegetable-origin powder, such as soybean powder and wheat flour, mineral fine powder, such as diatomaceous earth, apatite, gypsum, talc, bentonite, pyrophyllite and clay, and organic or inorganic materials, such as sodium benzoate, urea and Glauber's salt, can be used for solid-type formulations. In case liquid-type formulations are required, a petroleum fraction, such as kerosine, xylene and solvent naphtha, cyclohexane, cyclohexanone, DMF, DMSO, alcohols, acetone, trichloroethylene, methyl isobutyl ketone, mineral oils, vegetable oils, water, etc. can be used as a solvent. In order to assure uniform and stable physicochemical properties for such formulations, a surface active agent may be used, if appropriate. As examples for the surface active agent, whereas no limitation is given specifically thereto, nonionic surface active agents, such as alkylphenyl ethers added with polyoxyethylene, alkyl ethers added with polyoxyethylene, higher fatty acid esters added with polyoxyethylene, sorbitan higher fatty acid esters added with polyoxyethylene, and tristyrylphenyl ethers added with polyoxyethylene, alkylphenyl ether sulfates added with polyoxyethylene, alkylbenzenesulfonates, higher alcohol sulfates, alkylsulfates, alkylnaphthalene sulfonates, polycarboxylates, lignin sulfonates, formaldehyde condensates of alkylnaphthalene sulfonates, copolymers of isobutylene-maleic anhydride, etc. can be given.

The content of an active ingredient contained in the herbicide according to the present invention may be different and it may depend on each formulation types as described above. For example, such content may be in a range of from 5 to 90 % by weight (hereinafter describe as %), and preferably from 10 to 85%, for a wettable powder formulation; from 3 to 70%, and preferably from 5 to 60%, for an emulsifiable concentrate formulation; and from 0.01 to 50%, and preferably from 0.05 to 40%, for a granular formulation.

The wettable powder and emulsifiable concentrate formulations obtained as described above can be applied in a form of suspension or emulsion after diluting such formulations, respectively, with appropriate volume of water. The granule formulations obtained as described above can be directly applied to and incorporated into soil without dilution prior to or after germination of weeds. For practical applications of the herbicide according to the present invention, it is preferable to apply the formulation, wherein the compound of the present invention is contained as an active ingredient at an appropriate dose more than 0. 1 g/ha based on the active ingredient.

The herbicide according to the present invention may be also used as a mixture with any of other known fungicides, insecticides, acaricides, herbicides, plant growth regulators, fertilizers, etc. In particular, it is allowable to reduce the dose of the active ingredient contained in the inventive herbicide in practical uses by mixing with other herbicide. In this case, such mixing may enable not only to reduce labors required for weeding but also to give higher herbicidal performance because of a synergistic action brought by the herbicides mixed together. It is also possible to make combinations of the inventive herbicide and a plurality of other known herbicides.

For examples of the herbicides preferably associated with the inventive herbicide, anilide-type herbicides, such as difulfenican and propanil, chloroacetoanilide-type herbicides, such as alachlor and pretilachlor, aryloxyalcanic acid-type herbicides, such as 2,4-D and 2,4-DB, aryloxyphenoxyalcanic acid-type herbicides, such as dichlofopmethyl and phenoxaprop-ethyl, arylcarboxylic acid-type herbicides, such as dicamba and pyrithiobac, imidazilinone-type herbicides, such as imazaquin and imazethapyr, urea-type herbicides, such as diuron and isoproturon, carbamate-type herbicides, such as chlorpropham and phenmedipham, thiocarbamate-type herbicides, such as thiobencarb and EPTC, dinitroaniline-type herbicides, such as trifluralin and pendimethalin, diphenyl ether-type herbicides, such as acifluorfen and fomesafen, sulfonylurea-type herbicides, such as bensulfuron-methyl and nicosulfuron, triazinone-type herbicides, such as metribuzine and metamitron, triazine-type herbicides, such as atrazine and cyanazine, triazopyrimidine-type herbicides, such as flumetsulam, nitrile-type herbicides, such as bromoxynil and dichlobenil, phosphate-type herbicides, such as glyphosate and glyphosinate, quaternary ammonium salt-type herbicides, such as paraquat and difenzoquat, cyclic imide-type herbicides, such as flumiclorac-pentyl and fulthiacet-methyl, and others, such as isoxaben, ethofumesate, oxadiazon, quinclorac, cromazone, sulcotrion, cinmethyline, dithiopyr, pyrazolate, pyridate, fulpoxam, bentazon, benfulsate, and cyclohexanedione herbicides, such as sethoxydim and clethodim, can be given. In addition, vegetable oils and oil concentrates may be added to the combinations of the inventive herbicide and one or more of herbicides exemplified above.

## Best Modes for Carrying Out the Invention

Now, the present invention is further explained in detail with referring the following examples.

Example 1:
Manufacturing of 3-[2-chloro-3-(3-methylisoxazole-5-yl)-4-methylsulfonyl]benzoyl-1,6-dimethylpiperidine-2,4-dione (Compound No. I-8).

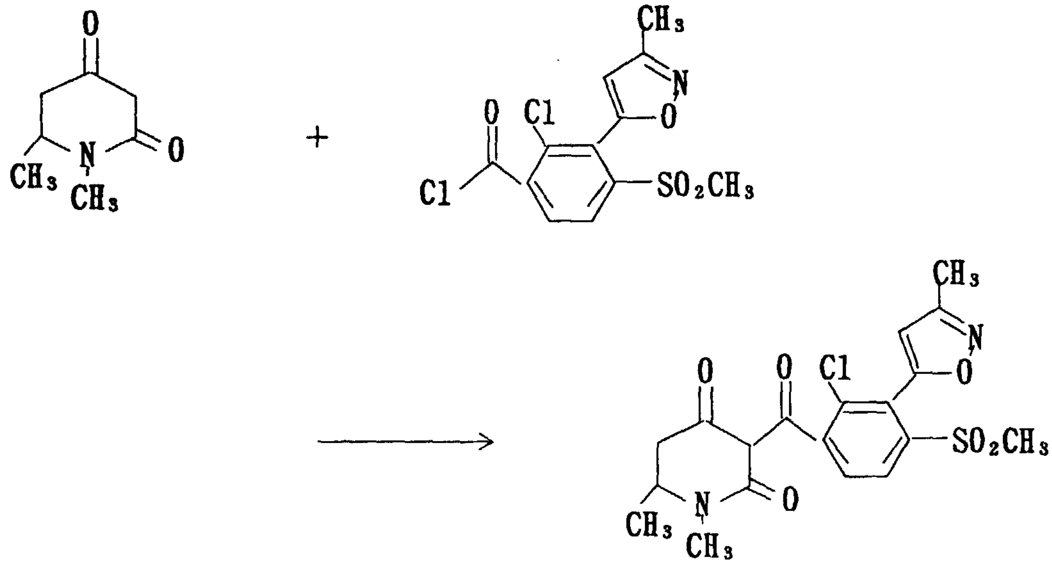

To 15 ml methylene chloride, were dissolved 1,6-dimethylpiperidine-2,4-dione in an amount of 1.4 g (0.010 mole) and triethylamine in an amount of 1.2 g (0.012 mole), and to the resulting solution, a solution prepared by dissolving under cooling with ice 2-chloro-3-(3-methylisoxazole-5-yl)-4-methylsulfonylbenzoyl chloride in an amount of 3. 18 g in 15 ml methylene chloride was fed dropwise. Following to the drop, the mixture was further allowed to a reaction under a room temperature. The reacted-solution was then washed with water and dried with anhydrous magnesium sulfate. The reacted-solution was further allowed to distillation for removing methylene chloride therefrom under reduced pressure to obtain o-benzoyl form in an amount of 3.0 g. Then, the o-benzoyl form obtained was dissolved in 20 ml acetonitrile without subjecting it to purification, added with triethylamine in an amount of 0.86 g (0.0085 mole) and then with acetonecyanohydrine in an amount of 0.10 g (0.0012 mole), and the resulting mixture was allowed to a reaction for 10 hours under a room temperature. The reacted-solution was poured into ice water, acidified with diluted hydrochloric acid and then subjected to an extraction with 100 ml chloroform. The organic-layer obtained was washed with water and saturated saline solution in turn and dried with anhydrous magnesium sulfate. After removing chloroform from the layer by distillation under reduced pressure, the residue obtained was purified by using silica gel column chromatography, where chloroform is used as an eluate, to obtain an objective compound in amorphous form in an amount of 1.2 g.

Example 2:
Manufacturing of 3-[2-chloro-3-(3-methylisoxazole-5-yl)-4-methylsulfonyl]benzoyl-5-bromo-1,6-dimethylpiperidine-2,4-dione (Compound No. I-12)

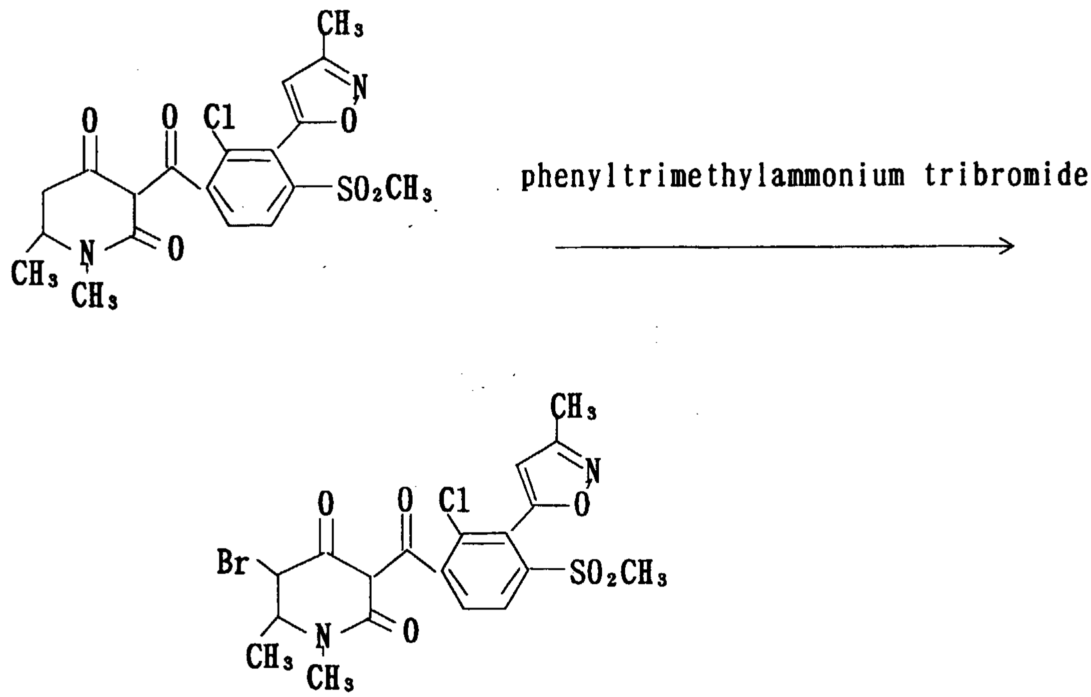


To 5 ml methylene chloride, was dissolved 3-[2-chloro-3-(3-methylisoxazole-5-yl)-4-methylsulfonyl]benzoyl-1,6-dimethylpiperidine-2,4-dione in an amount of 0.42 g (0.0010 mole), and the resulting solution was then added with phenyltrimethylammonium tribromide in an amount of 0.41 g (0.0011 mole) to proceed a reaction for 12 hours under a room temperature. The reacted-solution was then washed with water, dried with anhydrous magnesium sulfate and subjected to distillation for removing methylene chloride therefrom to obtain an objective compound in amorphous form in an amount of 0.40 g.

Example 3:
Manufacturing of 3-[2-chloro-3-(3-methylisoxazole-5-yl)-4-methylsulfonyl]benzoyl-1,6-dimethylpiperidine-5-ene-2,4-dione (Compound No. III-10)

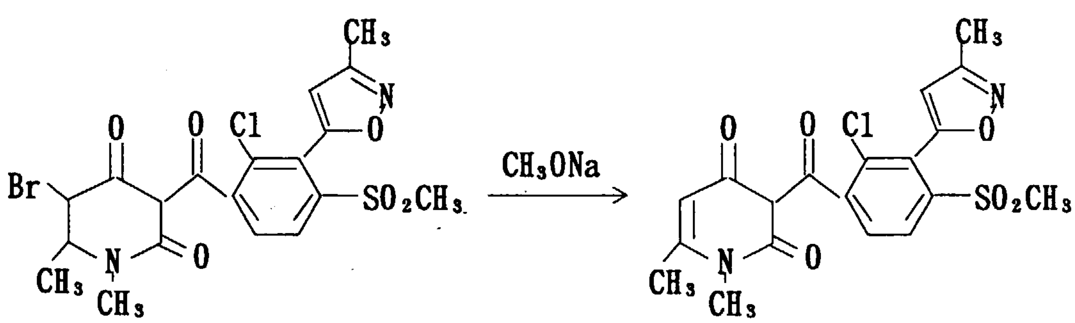

To 5 ml methanol, was dissolved 3-[2-chloro-3-(3-methylisoxazole-5-yl)-4-methylsulfonyl]benzoyl-5-bromo-1,6-dimethylpiperidine-2,4-dione in an amount of 0.40 g (0.00080 mole), and the resulting solution was then added with 28% methanol solution of sodium methylate in an amount of 0.50 g (0.0026 mole) to proceed a reaction for 3 hours under a room temperature. The reacted-solution was poured into ice water, acidified with diluted hydrochloric acid and then subjected to an extraction with 50 ml chloroform. The organic-layer obtained was washed with water and dried with anhydrous magnesium sulfate. After removing chloroform from the layer by distillation under reduced pressure, the residue obtained was purified by using a preparative thin-layer chromatography (chloroform : methanol = 30:1) to obtain an objective compound in amorphous form in an amount of 0.11 g.

Example 4:
Manufacturing of 3-[2-chloro-3-(3-methylisoxazole-5-yl)-4-methylsulfonyl]benzoyl-6-hydroxymethyl-1-methylpiperidine-2,4-dione (Compound No. I-14)

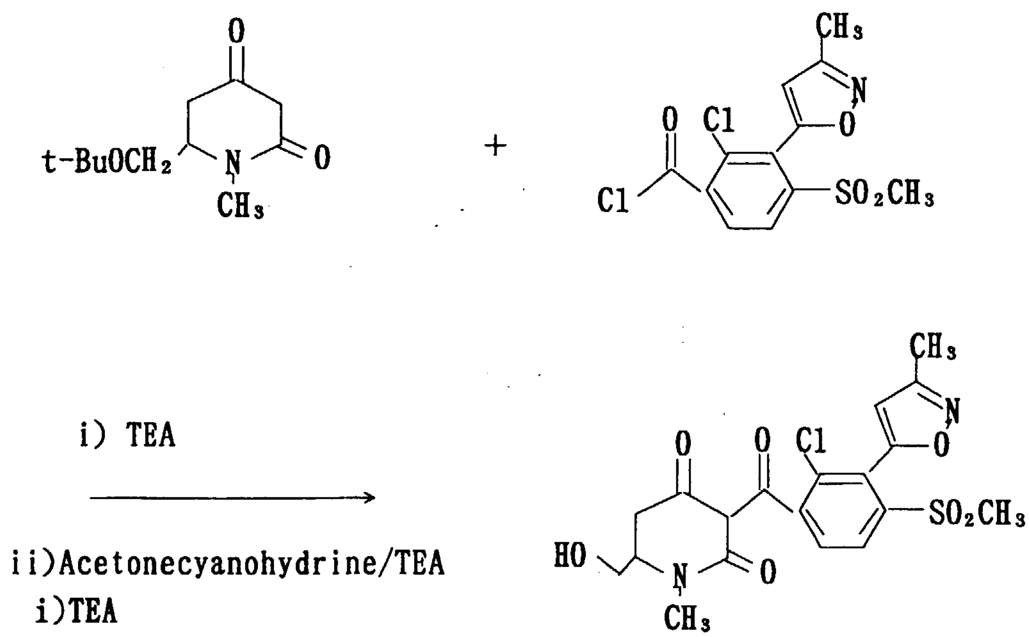


To 5 ml acetonitrile, were dissolved 6-tert-butoxymethyl-1-methylpiperidine-2,4-dione in an amount of 0.72 g (0.034 mole) and triethylamine in an amount of 0.52 g (0.0051 mole), and to the resulting solution, a solution prepared by dissolving 2-chloro-3-(3-methylisoxazole-5-yl)-4-methylsulfonylbenzoyl chloride in an amount of 1.06 g (0.0033 mole) in 5 ml acetonitrile was fed dropwise under cooling with ice. Following to the drop, the mixture was allowed to a reaction for 1 hour under a room temperature. The reacted-solution was then added with triethylamine in an amount of 0.52 g (0.0051 mole) and acetonecyanohydrine in an amount of 0.05 g (0.0006 mole), and the resulting mixture was allowed to a reaction for 5 hours under a room temperature. The reacted-solution was poured into ice water, acidified with diluted hydrochloric acid and subjected to an extraction with 50 ml chloroform. The organic-layer was washed with water and saturated saline solution in turn and dried with anhydrous magnesium sulfate. Following to removing chloroform by distillation under reduced pressure, the residue obtained was dissolved in a mixture of 10 ml ethanol and 5 ml concentrated sulfuric acid and the mixture was allowed to a reaction for 3 hours under reflux and heating. The reacted-solution was poured into ice water and then subjected to an extraction with 50 ml chloroform. The organic-layer obtained was washed with water and saturated saline solution in turn, dried with anhydrous magnesium sulfate and subjected to distillation for removing chloroform therefrom to obtain an objective compound in amorphous from in an amount of 1.20 g.

Example 5:
Manufacturing of 3-[2-chloro-3-(3-methylisoxazole-5-yl)-4-methylsulfonyl]benzoyl-6-iodomethyl-1-methylpiperidine-2,4-dione (Compound No. I-33)

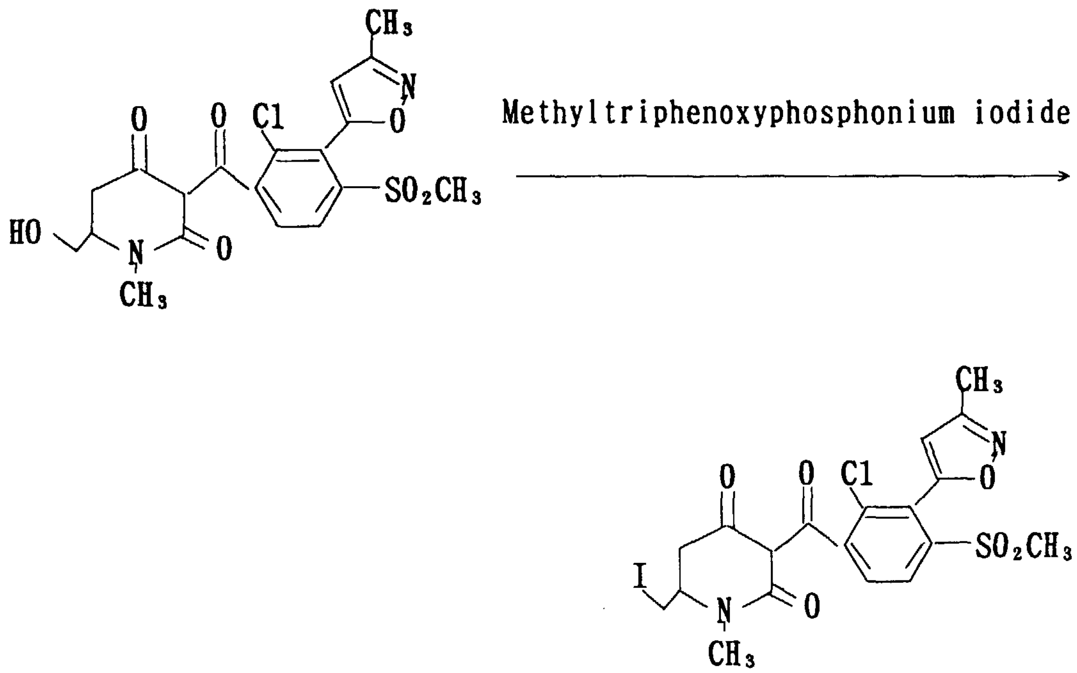


To 10 ml DMF, were dissolved 3-[2-chloro-3-(3-methylisoxazole-5-yl) -4-methylsulfonyl]benzoyl-6-hydroxymethyl-1-methylpiperidine-2,4-dione in an amount of 1.20 g (0.0027 mole) and methyltriphenoxyphosphonium iodide in an amount of 1.60 g (0.0035 mole), and the resulting solution was allowed to a reaction for 12 hours under a room temperature. The reacted-solution was poured into water and then subjected to an extraction with 50 ml chloroform. The organic-layer obtained was washed with water and saturated saline solution in turn, dried with anhydrous magnesium sulfate and subjected to distillation for removing chloroform therefrom under reduced pressure to obtain a residue. The residue was then purified by using silica gel column chromatography (chloroform : methanol = 20:1) to obtain an objective compound in amorphous form in an amount of 0.65 g.

Example 6:
Manufacturing of 4-[2-chloro-3-(3-methylisoxazole-5-yl)-4-methylsulfonyl]benzoyl-2-methyl-2-azabicyclo[4,1,0]heptane-3,5-dione (Compound No. IV-4)

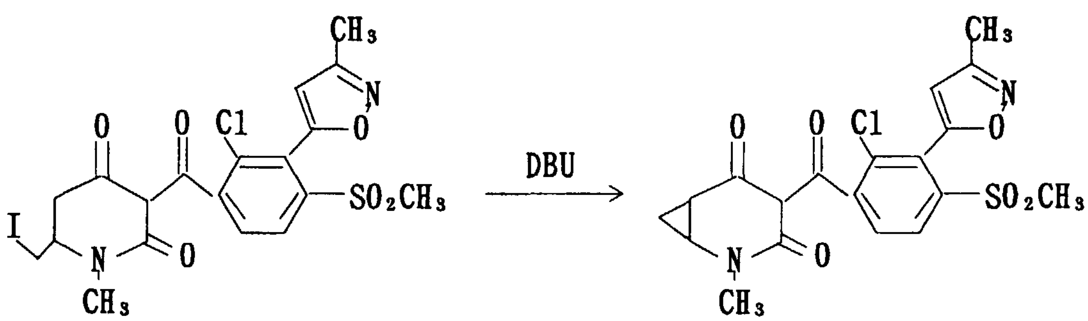

To 5 ml benzene, was added 3-[2-chloro-3-(3-methylisoxazole-5-yl)-4-methylsulfonyl]benzoyl-6-iodomethyl-1-methylpiperidine-2,4-dione in an amount of 0.65 g (0.0012 mole), and the resulting mixture was allowed to a reaction following to a further addition of DBU in an amount of 0.40 g (0.0026 mole) thereto for 12 hours under a room temperature. The reacted-solution was poured into ice water, acidified with diluted hydrochloric acid and subjected to an extraction with 50 ml chloroform. The organic-layer obtained was washed with water and saturated saline solution in turn, dried with anhydrous magnesium sulfate and subjected to distillation for removing chloroform therefrom under reduced pressure to obtain a residue. The residue was then purified by using thin-layer chromatography to obtain an objective compound in amorphous form in an amount of 0.10 g.

Now, the representative examples for the compound according to the present invention are presented in Tables 1, 2, 3 and 4 in the following.

In the tables from 1 to 4, heterocyclic groups are represented by symbols, such as A through N.

A : 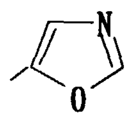

B : 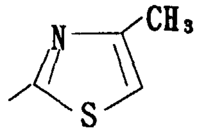

C : 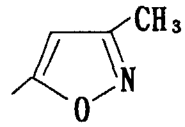

D : 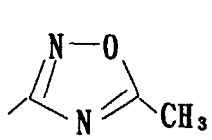

E : 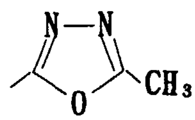

F : 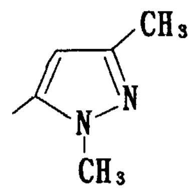

G : 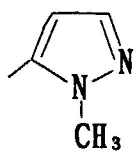

H : 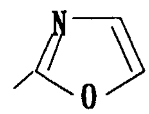

I : 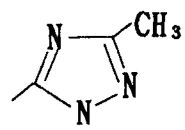

J : 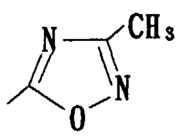

K : 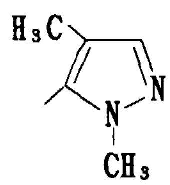

L : 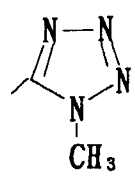

M : 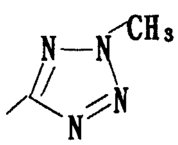

N : 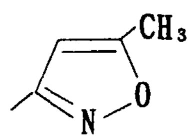

In Table 5, NMR data of the inventive compounds shown in Tables from 1 to 4 are described.

Table 1

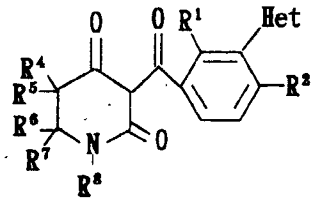

| No. | $R^1$ | $R^2$ | $R^4$ | $R^5$ | $R^6$ | $R^7$ | $R^8$ | Het | Physical Data |
|---|---|---|---|---|---|---|---|---|---|
| I-1 | Cl | Cl | H | H | $CH_3$ | H | H | A | |
| I-2 | Cl | $SO_2CH_3$ | H | H | $CH_3$ | H | H | A | |
| I-3 | Cl | Cl | H | H | $CH_3$ | H | H | B | |
| I-4 | Cl | $SO_2CH_3$ | H | H | $CH_3$ | H | H | B | |
| I-5 | Cl | Cl | H | H | $CH_3$ | H | H | C | |
| I-6 | Cl | $SO_2CH_3$ | H | H | $CH_3$ | H | H | C | * |
| 1-7 | Cl | Cl | H | H | $CH_3$ | H | $CH_3$ | C | |
| I-8 | Cl | $SO_2CH_3$ | H | H | $CH_3$ | H | $CH_3$ | C | * |
| I-9 | Cl | Cl | $CH_3$ | H | H | H | $CH_3$ | C | |
| I-10 | Cl | $SO_2CH_3$ | $CH_3$ | H | H | H | $CH_3$ | C | |
| I-11 | Cl | Cl | Br | H | $CH_3$ | H | $CH_3$ | C | |
| I-12 | Cl | $SO_2CH_3$ | Br | H | $CH_3$ | H | $CH_3$ | C | * |
| 1-13 | Cl | $SO_2CH_3$ | H | H | H | $CH_2OC(O)CH_3$ | $CH_3$ | C | * |
| I-14 | Cl | $SO_2CH_3$ | H | H | H | $CH_2OH$ | $CH_3$ | C | * |
| I-15 | Cl | $SO_2CH_3$ | H | H | $CH_3$ | H | H | D | |
| I-16 | Cl | $SO_2CH_3$ | H | H | $CH_3$ | H | H | E | |

Note: In the column indicated as Physical Data, [ ] means a melting point (℃), and * mark shows that the compound has NMR data and is presented in Table 5. This explanation is also applicable for the following tables.

Table 1 (Continued)

| No. | $R^1$ | $R^2$ | $R^4$ | $R^5$ | $R^6$ | $R^7$ | $R^8$ | Het | Physical Data |
|---|---|---|---|---|---|---|---|---|---|
| I-17 | Cl | Cl | H | H | $CH_3$ | H | H | F | |
| I-18 | Cl | $SO_2CH_3$ | H | H | $CH_3$ | H | H | F | |
| I-19 | Cl | Cl | H | H | $CH_3$ | H | $CH_3$ | H | * |
| I-20 | Cl | $SO_2CH_3$ | H | H | $CH_3$ | H | $CH_3$ | H | * |
| I-21 | Cl | Cl | H | H | $CH_3$ | H | $CH_3$ | G | * |
| I-22 | Cl | $SO_2CH_3$ | H | H | $CH_3$ | H | $CH_3$ | G | * |
| I-23 | Cl | Cl | H | H | $CH_3$ | H | $CH_3$ | I | * |
| I-24 | Cl | $SO_2CH_3$ | H | H | $CH_3$ | H | $CH_3$ | I | * |
| I-25 | Cl | Cl | H | H | $CH_3$ | H | $CH_3$ | J | * |
| I-26 | Cl | $SO_2CH_3$ | H | H | $CH_3$ | H | $CH_3$ | J | * |
| I-27 | Cl | Cl | H | H | $CH_3$ | H | $CH_3$ | K | * |
| I-28 | Cl | $SO_2CH_3$ | H | H | $CH_3$ | H | H | K | |
| I-29 | Cl | Cl | H | H | $CH_3$ | H | $CH_3$ | L | * |
| I-30 | Cl | $SO_2CH_3$ | H | H | $CH_3$ | H | $CH_3$ | L | [210-213 ] |
| I-31 | Cl | $SO_2CH_3$ | H | H | $CH_3$ | H | H | M | |
| I-32 | Cl | $SO_2CH_3$ | H | H | $CH_3$ | H | H | N | |
| I-33 | Cl | $SO_2CH_3$ | H | H | H | $CH_2I$ | $CH_3$ | C | amorphous |

Table 2

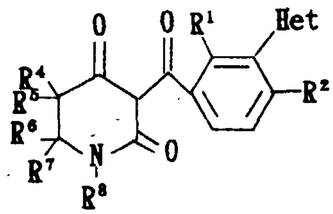

| No. | $R^1$ | $R^2$ | $R^4$ | $R^5$ | $R^6$ | $R^7$ | $R^8$ | Het | Physical Data |
|---|---|---|---|---|---|---|---|---|---|
| II-1 | $CH_3$ | $SO_2CH_3$ | H | H | $CH_3$ | H | $CH_3$ | A | |
| II-2 | $OCH_3$ | $SO_2CH_3$ | H | H | $CH_3$ | H | $CH_3$ | A | |
| II-3 | $CH_3$ | $SO_2CH_3$ | H | H | $CH_3$ | H | H | C | * |
| II-4 | $OCH_3$ | $SO_2CH_3$ | H | H | $CH_3$ | H | H | C | * |
| II-5 | H | $SO_2CH_3$ | H | H | $CH_3$ | H | $CH_3$ | C | * |
| II-6 | $CH_3$ | $SO_2CH_3$ | H | H | $CH_3$ | H | $CH_3$ | C | |
| II-7 | $OCH_3$ | $SO_2CH_3$ | H | H | $CH_3$ | H | $CH_3$ | C | |
| II-8 | $CH_3$ | $SO_2CH_3$ | H | H | $CH_3$ | H | $CH_3$ | F | |
| II-9 | $OCH_3$ | $SO_2CH_3$ | H | H | $CH_3$ | H | $CH_3$ | F | |
| II-10 | $CH_3$ | $SO_2CH_3$ | H | H | $CH_3$ | H | $CH_3$ | H | |
| II-11 | $OCH_3$ | $SO_2CH_3$ | H | H | $CH_3$ | H | $CH_3$ | H | |
| II-12 | $CH_3$ | $SO_2CH_3$ | H | H | $CH_3$ | H | $CH_3$ | I | |
| II-13 | $OCH_3$ | $SO_2CH_3$ | H | H | $CH_3$ | H | $CH_3$ | I | |
| II-14 | $CH_3$ | $SO_2CH_3$ | H | H | $CH_3$ | H | $CH_3$ | M | |
| II-15 | $OCH_3$ | $SO_2CH_3$ | H | H | $CH_3$ | H | $CH_3$ | M | |
| II-16 | $CH_3$ | $SO_2CH_3$ | H | H | $CH_3$ | H | $CH_3$ | N | |
| II-17 | $OCH_3$ | $SO_2CH_3$ | H | H | $CH_3$ | H | $CH_3$ | N | |

Table 3

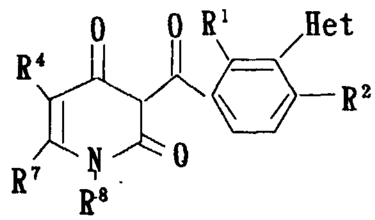

| No. | $R^1$ | $R^2$ | $R^4$ | $R^7$ | $R^8$ | Het | Physical Data |
|---|---|---|---|---|---|---|---|
| III-1 | Cl | $SO_2CH_3$ | H | $CH_3$ | H | A | |
| III-2 | Cl | $SO_2CH_3$ | H | $CH_3$ | $CH_3$ | A | |
| III-3 | $CH_3$ | $SO_2CH_3$ | H | $CH_3$ | H | A | |
| III-4 | $OCH_3$ | $SO_2CH_3$ | H | $CH_3$ | H | A | |
| III-5 | Cl | Cl | H | H | H | C | |
| III-6 | Cl | $SO_2CH_3$ | H | H | H | C | |
| III-7 | Cl | Cl | H | $CH_3$ | H | C | |
| III-8 | Cl | $SO_2CH_3$ | H | $CH_3$ | H | C | [270 ℃ up ] |
| III-9 | Cl | Cl | H | $CH_3$ | $CH_3$ | C | |
| III-10 | Cl | $SO_2CH_3$ | H | $CH_3$ | $CH_3$ | C | * |
| III-11 | $CH_3$ | $SO_2CH_3$ | H | H | H | C | |
| III-12 | $CH_3$ | $SO_2CH_3$ | H | $CH_3$ | H | C | |
| III-13 | $OCH_3$ | $SO_2CH_3$ | H | H | H | C | |
| III-14 | $OCH_3$ | $SO_2CH_3$ | H | $CH_3$ | H | C | |
| III-15 | Cl | Cl | Br | $CH_3$ | H | F | [223-225 ] |
| III-16 | Cl | Cl | H | $CH_3$ | $CH_3$ | F | * |
| III-17 | Cl | $SO_2CH_3$ | H | $CH_3$ | H | F | |

Table 3 (Continued)

| No. | $R^1$ | $R^2$ | $R^4$ | $R^7$ | $R^8$ | Het | Physical Data |
|---|---|---|---|---|---|---|---|
| III-18 | $CH_3$ | $SO_2CH_3$ | H | $CH_3$ | H | F | |
| III-19 | $OCH_3$ | $SO_2CH_3$ | H | $CH_3$ | H | F | |
| III-20 | Cl | $SO_2CH_3$ | H | $CH_3$ | H | H | |
| III-21 | $CH_3$ | $SO_2CH_3$ | H | $CH_3$ | H | H | |
| III-22 | $OCH_3$ | $SO_2CH_3$ | H | $CH_3$ | H | H | |
| III-23 | Cl | $SO_2CH_3$ | H | $CH_3$ | H | M | |
| III-24 | $CH_3$ | $SO_2CH_3$ | H | $CH_3$ | H | M | |
| III-25 | $OCH_3$ | $SO_2CH_3$ | H | $CH_3$ | H | M | |
| III-26 | Cl | $SO_2CH_3$ | H | $CH_3$ | H | N | |
| III-27 | $CH_3$ | $SO_2CH_3$ | H | $CH_3$ | H | N | |
| III-28 | $OCH_3$ | $SO_2CH_3$ | H | $CH_3$ | H | N | |

Table 4

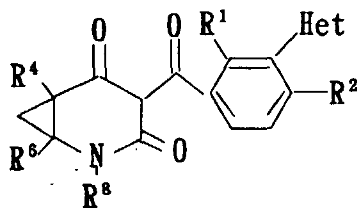

| No. | $R^1$ | $R^2$ | $R^4$ | $R^6$ | $R^8$ | Het | Physical Data |
|---|---|---|---|---|---|---|---|
| IV-1 | Cl | $SO_2CH_3$ | H | H | $CH_3$ | A | |
| IV-2 | $CH_3$ | $SO_2CH_3$ | H | H | $CH_3$ | A | |
| IV-3 | $OCH_3$ | $SO_2CH_3$ | H | H | $CH_3$ | A | |
| IV-4 | Cl | $SO_2CH_3$ | H | H | $CH_3$ | C | * |
| IV-5 | $CH_3$ | $SO_2CH_3$ | H | H | $CH_3$ | C | [193-194 ] |
| IV-6 | $OCH_3$ | $SO_2CH_3$ | H | H | $CH_3$ | C | |
| IV-7 | Cl | $SO_2CH_3$ | H | H | $CH_3$ | F | |
| IV-8 | $CH_3$ | $SO_2CH_3$ | H | H | $CH_3$ | F | |
| IV-9 | $OCH_3$ | $SO_2CH_3$ | H | H | $CH_3$ | F | |
| IV-10 | Cl | $SO_2CH_3$ | H | H | $CH_3$ | H | |
| IV-11 | $CH_3$ | $SO_2CH_3$ | H | H | $CH_3$ | H | |
| IV-12 | $OCH_3$ | $SO_2CH_3$ | H | H | $CH_3$ | H | |
| IV-13 | Cl | $SO_2CH_3$ | H | H | $CH_3$ | M | |
| IV-14 | $CH_3$ | $SO_2CH_3$ | H | H | $CH_3$ | M | |
| IV-15 | $OCH_3$ | $SO_2CH_3$ | H | H | $CH_3$ | M | |
| IV-16 | Cl | $SO_2CH_3$ | H | H | $CH_3$ | N | |
| IV-17 | $CH_3$ | $SO_2CH_3$ | H | H | $CH_3$ | N | |
| IV-18 | $OCH_3$ | $SO_2CH_3$ | H | H | $CH_3$ | N | |

Table 5

| Compound No. | NMR Data ($CDCl_3$, ppm) |
|---|---|
| I-6 | 1.33(s, 3H), 2.43(s, 3H), 2.55-2.88(m, 2H), 3.08(s, 3H), 3.85(m, 1H), 6.35(s, 1H), 6.48(m, 1H), 7.55(m, 1H), 8.20(m, 1H), 16.65, 17.65(1H) |
| I-8 | 1.30(m, 3H), 2.31(m, 1H), 2.41(s, 3H), 2.87(m, 1H), 3.16(s, 3H), 3.28 (s, 3H), 3.72(m, 1H), 6.47(m, 1H), 7.52(m, 1H), 8.18(m, 1H), 18.20(m, 1H) |
| I-12 | 1.52(s, 3H), 2.42(s, 3H), 3.08(s, 3H), 3.07, 3.22(1H), 3.28(s, 3H), 4.18(m, 1H), 6.50(m, 1H), 7.53(m, 1H), 8.22(m, 1H), 18.20-18.42(m, 1H) |
| I-13 | 2.03(m, 3H), 2.42(s, 3H), 2.45(m, 1H), 2.90(m, 1H), 3.06(s, 3H), 3.22(s, 3H), 3.84(m, 1H), 4.20(m, 2H), 6.46(m, 1H), 7.50(m, 1H), 8.19(m, 1H), 18.18(s, 1H) |
| I-14 | 2.38(m, 1H), 2.42(m, 3H), 2.82(s, 1H), 3.06(m, 3H), 3.21(s, 3H), 3.50-3.77(m, 3H), 6.45(m, 1H), 7.48(m, 1H), 8.15(m, 1H), 18.11(bs, 1H) |
| I-19 | 1.28(m, 3H), 2.31(m, 1H), 2.85(m, 1H), 3.15(s, 3H), 3.68(s, 1H), 7.31(m, 1H), 7.35(s, 1H), 7.46(m, 1H), 7.82(s, 1H), 18.20(1H) |
| I-20 | 1.30(m, 3H), 2.32(m, 1H), 2.85(m, 1H), 3.18(s, 3H), 3.30(s, 3H), 3.70(m, 1H), 7.32(S, 1H), 7.55(m, 1H), 7.87(s, 1H), 8.12(m, 1H), 18.20(m, 1H) |
| I-21 | 1.28(m, 3H), 2.30(m, 1H), 2.83(m, 1H), 3.15(s, 3H), 3.62-3.88(m, 4H), 6.25-6.40(m, 1H), 7.25(m, 1H), 7.47(m, 1H), 7.66(s, 1H), 18.25(s, 1H) |
| I-22 | 1.30(m, 3H), 2.29(m, 1H), 2.86, 2.95(s, 3H), 2.80-3.02(m, 1H), 3.18(s 3H)3.62-3.77(s, 4H), 6.37(m, 1H), 7.50(m, 1H), 7.59(s, 1H), 8.21(m, 1H), 18.22 (m, 1H) |
| I-23 | 1.25(m, 3H), 2.26(m, 1H), 2.43(s, 3H), 2.82(m, 1H), 3.12(s, 3H), 3.60-3.75(m, 4H), 7.28(m, 1H), 7.46(m, 1H), 18.23(1H) |
| I-24 | 1.27(m, 3H), 2.27(m, 1H), 2.42(s, 3H), 2.83(m, 1H), 3.16(s, 3H), 3.26(s, 3H), 3.59-3.75(m, 4H), 7.54(m, 1H), 8.16(m, 1H), 18.22(1H) |
| I-25 | 1.28(m, 3H), 2.30(m, 1H), 2.53(s, 3H), 2.85(m, 1H), 3.16(s, 3H), 3.70(m, 1H), 7.30-7.45(m, 1H), 7.48(m, 1H), 18.21(1H) |
| I-26 | 1.30(s, 3H), 2.30(m, 1H), 2.52(s, 3H), 2.85(m, 1H), 3.18(s, 3H), 3.25(s, 3H), 3.72(m, 1H), 7.60(m, 1H), 8.15(m, 1H), 18.15(s, 1H) |
| I-27 | 1.28(s, 3H), 1.93(s, 3H), 2.30(m, 1H), 2.84(m, 1H), 3.15(S, 3H), 3.65(s, 3H), 3.70(m, 1H), 7.25(m, 1H), 7.40(s, 1H), 7.50(m, 1H), 18.30(s, 1H) |
| I-29 | 1.30(d, 3H), 2.30(m, 3H), 2.90(m, 1H), 3.18(S, 3H), 3.72(m, 1H), 3.97(s, 1H), 7.43(m, 1H), 7.56(m, 1H) |
| II-3 | 1.28(m, 3H), 2.10(m, 3H), 2.40(s, 3H), 2.50-2.75(m, 2H), 2.95(s, 3H), 3.80(m, 1H), 6.40(m, 1H), 7.44(m, 1H), 8.10(m, 1H) |
| II-4 | 1.33(m, 3H), 2.38(s, 3H), 2.65(m, 2H), 2.92(s, 3H), 3.40(s, 3H), 3.85(m, 1H), 6.41 (m, 1H), 7.40 (m, 1H), 8.15(m, 1H) |
| II-5 | 1.32(s, 3H), 2.32(m, 1H), 2.38(s, 3H), 2.90(m, 1H), 3.16(s, 3H), 3.18(s, 3H), 3.72(m, 1H), 6.61(s, 1H), 7.72(m, 2H), 8.23(m, 1H), 18.49(s, 1H) |
| III-8 | 1.95, 2.18(s, 3H), 2.22(s, 3H), 2.91(s, 3H), 5.67(s, 1H), 6.28(s, 1H), 7.33(m, 1H), 8.00(m, 1H), 11.49, 11.92(1H), 14.15, 15.12(1H) |
| III-10 | 2.39(s, 3H), 2.41(s, 3H), 3.07(s, 3H), 3.34(s, 3H), 5.96(s, 1H), 6.48(s, 1H), 7.47(m, 1H), 8.19(m, 1H), 14.25, 15.28(1H) |
| III-15 | 1.98(s, 3H), 2.31(s, 3H), 3.63(s, 3H), 5.87(s, 1H), 7.28(m, 1H), 7.47(m, 1H), 11.90(bs, 1H), 14.42(1H) |
| III-16 | 2.30(s, 3H), 2.38(s, 3H), 3.32(s, 3H), 5.92(s, 1H), 7.27(m, 2H), 9.52(m, 1H), 14.45, 15.57(1H) |

Table 5 (continued)

| Compound No. | NMR Data ($CDCl_3$, ppm) |
|---|---|
| IV-4 | 1.05(m, 1H), 1.52(m, 1H), 2.03(m, 1H), 2.41(s, 3H), 3.07(s, 3H), 3.30(s, 3H), 6.47(m, 1H), 7.37-7.60(m, 1H), 8.16(m, 1H), 18.26-18.38(m, 1H) |

Now, examples for formulations suitable for the herbicide according to present invention are given hereinbelow. However, the type of an active ingredient and, types and adding rates of additives to be contained in such formulations shall be varied in wide range and shall not be limited to the ones specified in the examples described below. "Parts" described in the following Formulation Examples means "parts by weight".

Formulation Example 7 : Wettable powder

| | |
|---|---|
| The inventive compound | 20 parts |
| White carbon | 20 parts |
| Diatomaceous earth | 52 parts |
| Sodium alkylsulfate | 8 parts |

All materials are uniformly mixed and ground to fine powder to obtain a wettable powder formulation which contains an active ingredient at a rate of 20% by weight.

Formulation Example 8 : Emulsifiable Concentrate

| | |
|---|---|
| The inventive compound | 20 parts |
| Xylene | 55 parts |
| Dimethylformamide | 15 parts |
| Polyoxyethylenephenyl ether | 10 parts |

All materials are mixed and dissolved to obtain an emulsifiable concentrate formulation which contains an active ingredient at a rate of 20%.

Formulation Example 9 : Granular Formulation

| | |
|---|---|
| The inventive compound | 5 parts |
| Talc | 40 parts |
| Clay | 38 parts |
| Bentonite | 10 parts |
| Sodium alkylsulfate | 7 parts |

All materials are uniformly mixed, ground to fine powder and granulated into granules each having a diameter of from 0.5 to 1.0 mm to obtain a granular formulation which contains an active ingredient at a rate of 5% by weight.

Test examples being carried out for presenting herbicidal activity of the herbicides according to the present invention are now described hereinbelow.

Herbicidal activity is evaluated pursuant to the following criterion, and it is expressed as an index for weed control.

Criterion for Assessment

| % of Weeds control | Index for weed control |
|---|---|
| 0 % | 0 |
| 20 - 29 % | 2 |
| 40 - 49 % | 4 |
| 60 - 69 % | 6 |
| 80 - 89 % | 8 |
| 100 % | 10 |

Indexes 1, 3, 5, 7 and 9 each represents an intermediate activity between 0 and 2, 2 and 4, 4 and 6, 6 and 8, and 8 and 10, respectively.

% of Weeds control = [(Fresh weight of shoots in untreated plot - Fresh weight of shoots in a treated-plot) $\div$ (Fresh weight of shoots in untreated plot)] $\times$ 100

Test Example 1 : Foliar Application in Upland crops:

In a 200 $cm^2$ planting pot filled with soil, seeds of velvetleaf, barnyardglass, common cocklebur, giant foxtail, and maize are planted on the surface of soil, respectively, and are then covered with slight amount of soil to grow in a greenhouse. When each weed has grown to 5 to 25 cm height, respectively, emulsions adjusted at fixed concentrations presented in the example 8 by diluting with water and prepared from the emulsifiable concentrate formulations of each test compounds were sprayed to shoots of the weeds, respectively, at a dose rate of 250 g/ha by using a small sprayer. 3 weeks later, herbicidal performance was checked, respectively, and the results are presented in Table 6.

Table 6

| Compound No. | Dose (g/ha) | Index for Weed control | | | | |
|---|---|---|---|---|---|---|
| | | Velvet leaf | Barnyard glass | common cocklebur | giant foxtail | Maize |
| I- 6 | 250 | 10 | 9 | 9 | 9 | 0 |
| I- 8 | 250 | 10 | 10 | 10 | 10 | 0 |
| I-12 | 250 | 9 | 9 | 9 | 9 | 0 |
| I-26 | 250 | 10 | 9 | 10 | 10 | 2 |
| III- 8 | 250 | 9 | 9 | 9 | 9 | 0 |
| III-10 | 250 | 9 | 6 | 8 | 8 | 0 |
| IV- 4 | 250 | 10 | 10 | 10 | 9 | 4 |

Industrial Use of the Invention

As described above, the compounds specified in the present invention can be advantageously manufactured in an industrial scale and are useful in agricultural and chemical industries as an active ingredient of herbicides for controlling weeds grown in the fields of maize plants.

## Claims

1. Substituted piperidinedione derivatives represented by a general formula (I) or salts thereof;

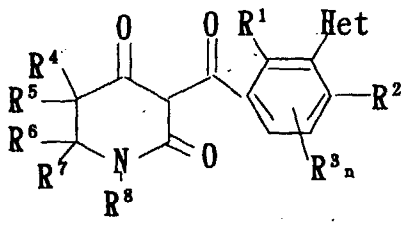

(1)

wherein $R^1$ represents nitro, cyano, halogeno, $C_{1-6}$ alkyl, $C_{1-6}$ alkoxy, $C_{1-6}$ haloalkyl, $C_{1-6}$ alkylthio, $C_{1-6}$ alkylsulfinyl or $C_{1-6}$ alkylsulfonyl;
$R^2$ represents nitro, cyano, halogeno, $C_{1-6}$ alkyl, $C_{1-6}$ alkoxy, $C_{1-6}$ haloalkyl, $C_{1-6}$ haloalkoxy, $C_{1-6}$ alkylthio, $C_{1-6}$ alkylsulfinyl or $C_{1-6}$ alkylsulfonyl;
$R^3$ represents nitro, cyano, halogeno, $C_{1-6}$ alkyl, $C_{1-6}$ alkoxy, $C_{1-6}$ haloalkyl, $C_{1-6}$ alkylthio, $C_{1-6}$ alkylsulfinyl or $C_{1-6}$ alkylsulfonyl;
n is 0, 1 or 2;
$R^4$, $R^5$, $R^6$ and $R^7$ each independently represents hydrogen, halogeno, $C_{1-6}$ alkyl, $C_{1-6}$ haloalkyl, $C_{1-6}$ alkoxy $C_{1-6}$ alkyl, $C_{1-6}$ alkylcarbonyloxy $C_{1-6}$ alkyl, or hydroxy $C_{1-6}$ alkyl; and $R^4$ and $R^5$, and $R^6$ and $R^7$ may form in together a $C_{2-5}$ alkylene chain, and further, $R^5$ and $R^6$ may form in together a either single bond or $C_{1-4}$ alkylene chain;
$R^8$ represents hydrogen, $C_{1-6}$ alkyl, $C_{2-6}$ alkenyl, $C_{2-6}$ alkynyl, $C_{1-6}$ haloalkyl, $C_{2-6}$ haloalkenyl, $C_{2-6}$ haloalkynyl, $C_{1-6}$ alkoxy, $C_{2-6}$ alkenyloxy, $C_{2-6}$ alkylnyloxy, $C_{1-6}$ haloalkoxy, $C_{2-6}$ haloalkenyloxy, or $C_{2-6}$ haloalkynyloxy;
Het represents a saturated or unsaturated 5-membered heterocycle substituted with $R^9$ or $R^{10}$ both containing 1-4 N, O or S atoms, and is bonded to the benzene ring of the formula (1) at the carbon atom, and
$R^9$ and $R^{10}$ each independently represents hydrogen, halogeno, $C_{1-6}$ alkyl or $C_{1-6}$ alkoxy.

2. The substituted piperidinedione derivatives represented by the general formula (1) or salts thereof according to claim 1, wherein Het is one selected from a group consisting of the following heterocyclic groups;

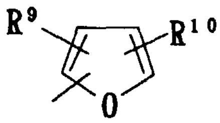 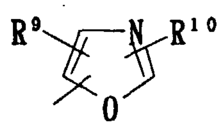 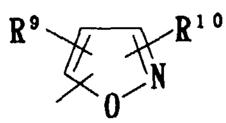

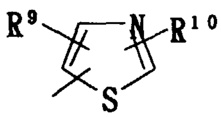 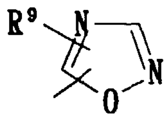 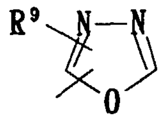

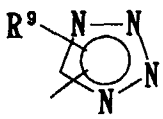 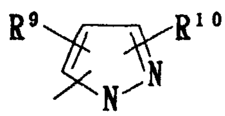 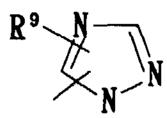

wherein $R^9$ and $R^{10}$ are as defined above.

3. Herbicides characterized by comprising one or more of the substituted-piperidinedione derivatives represented by the general formula (1) and/or salts thereof;

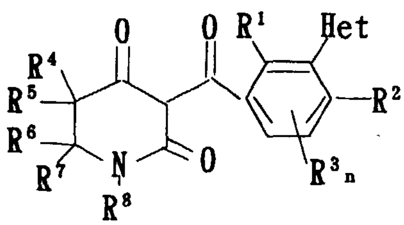

(1)

wherein $R^1$, $R^2$, $R^3$, $R^4$, $R^5$, $R^6$, $R^7$, $R^8$, n and Het are as defined above, as an active ingredient.

## INTERNATIONAL SEARCH REPORT

International application No.
PCT/JP97/04815

**A. CLASSIFICATION OF SUBJECT MATTER**

Int.Cl[6] C07D413/10, 417/10, 401/10, A01N43/40, 43/76, 43/80, 43/78, 43/836, 43/824, 43/713, 43/56, 43/653

According to International Patent Classification (IPC) or to both national classification and IPC

**B. FIELDS SEARCHED**

Minimum documentation searched (classification system followed by classification symbols)

Int.Cl[6] C07D413/00-413/14, 417/00-417/14, 401/00-401/14, A01N43/00-43/92

Documentation searched other than minimum documentation to the extent that such documents are included in the fields searched

Electronic data base consulted during the international search (name of data base and, where practicable, search terms used)

CA (STN), REGISTRY (STN), WPIDS (STN)

**C. DOCUMENTS CONSIDERED TO BE RELEVANT**

| Category* | Citation of document, with indication, where appropriate, of the relevant passages | Relevant to claim No. |
|---|---|---|
| P, A | WO, 97/46530, A1 (Du Pont De Nemours and Co. E.I.), December 11, 1997 (11. 12. 97), Full text (Family: none) | 1-3 |
| P, A | WO, 97/35851, A1 (Nippon Soda Co., Ltd.), October 2, 1997 (02. 10. 97), Full text & AU, 9719454, A | 1-3 |
| A | WO, 96/26200, A1 (BASF A.G.), August 29, 1996 (29. 08. 96), Full text & EP, 811005, A1 & AU, 9648753, A | 1-3 |
| A | JP, 55-122785, A (Yoshitomi Pharmaceutical Industries, Ltd.), September 20, 1980 (20. 09. 80), Full text (Family: none) | 1-3 |

☐ Further documents are listed in the continuation of Box C. ☐ See patent family annex.

* Special categories of cited documents:

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step when the document is taken alone

"Y" document of particular relevance; the claimed invention cannot be considered to involve an inventive step when the document is combined with one or more other such documents, such combination being obvious to a person skilled in the art

"&" document member of the same patent family

| | |
|---|---|
| Date of the actual completion of the international search<br>March 24, 1998 (24. 03. 98) | Date of mailing of the international search report<br>March 31, 1998 (31. 03. 98) |
| Name and mailing address of the ISA/<br>Japanese Patent Office | Authorized officer |
| Facsimile No. | Telephone No. |

Form PCT/ISA/210 (second sheet) (July 1992)